# EUROPÄISCHE PATENTSCHRIFT

(11) **EP 2 048 947 B1**
(45) Veröffentlichungstag und Bekanntmachung des Hinweises auf die Patenterteilung: **02.12.2009**
(21) Anmeldenummer: 07788223.1
(22) Anmeldetag: 03.08.2007
(51) Int. Cl.: A01N 25/32, A01N 37/10, A01N 43/78, A01N 43/80, A01N 43/56

(54) **WÄSSRIGE WIRKSTOFFKONZENTRATE MIT HERBIZIDER WIRKUNG**
AQUEOUS ACTIVE INGREDIENT CONCENTRATE HAVING AN HERBICIDAL EFFECT
CONCENTRÉS AQUEUX DE PRINCIPES ACTIFS À EFFET HERBICIDE

(30) Priorität: 04.08.2006 EP 06118443
(43) Veröffentlichungstag der Anmeldung: 22.04.2009
(73) Patentinhaber: BASF SE, 67056 Ludwigshafen (DE)
(72) Erfinder: BRATZ, Matthias, 67133 Maxdorf (DE); BERGHAUS, Rainer, 67346 Speyer (DE); STEINBRENNER, Ulrich, 67435 Neustadt (DE)
(86) Internationale Anmeldenummer: PCT/EP2007/058092
(87) Internationale Veröffentlichungsnummer: WO 2008/015280

(56) Entgegenhaltungen:
- WO-A-98/31681
- WO-A-99/65314
- WO-A-2005/087785
- WO-A-2006/003371
- WO-A-2007/059870
- US-A1- 2003 050 194
- GARST R.: "ALKYL POLYGLYCOSIDES TECHNOLOGY, PROPERTIES AND APPLICATION" 1997, VCH VERLAGGESELLSCHAFT , GERMANY , XP002480974 Seite 134, Absatz 5-7 - Seite 136 Seite 137, Absatz 9

## Beschreibung

Die vorliegende Erfindung betrifft wässrige Wirkstoffkonzentrate mit herbizider Wirkung, die in gelöster Form enthalten:
a) mindestens eine 4-Benzoyl-substituierte Pyrazolverbindung der Formel **I** 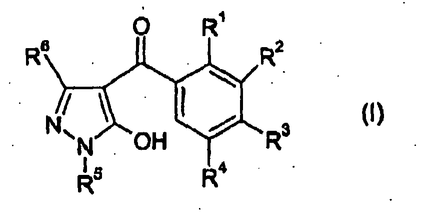 worin
   - R¹, R³: unabhängig voneinander für Wasserstoff, Halogen, Methyl, Halogenmethyl, Methoxy, Halogenmethoxy, Methylthio, Methylsulfinyl oder Methylsulfonyl stehen;
   - R²: für einen 5-gliedrigen heterocyclischen Rest steht, der unsubstituiert ist oder 1,2, 3 oder 4 Substituenten trägt, die unter Halogen, C₁-C₆-Alkyl. C₁-C₄-Akoxy. C₁-C₄-Halogenalkyl, C₁-C₄-Halogenalkoxy und C₁-C₄-Alkylthio ausgewählt sind;
   - R⁴: Wasserstoff, Halogen oder Methyl bedeutet;
   - R⁵: C₁C₆-Alkyl, C₃-C₆-Cycloalkyl oder C₃-C₆-Cycloalkylmethyl bedeutet; und
   - R⁶: für Wasserstoff oder C₁-C₄-Alkyl steht;
   oder eines seiner landwirtschaftlich verwendbaren Salze;
b) mindestens eine Benzoesäureverbindung der Formel **II** 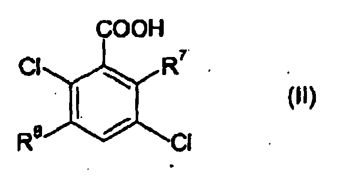 worin
   - R⁷: Wasserstoff, Halogen, Hydroxy oder Methoxy und
   - R⁸: Wasserstoff, Halogen oder Amino bedeuten;
   oder eines seiner landwirtschaftlich verwendbaren Salze; und
c) mindestens eine nichtionische oberflächenaktive Substanz S, ausgewählt unter Polyetherverbindungen, die von Ethylenoxid abgeleitete Wiederholungseinheiten aufweisen, Alkylpolyglykosiden und Gemischen davon
worin das Gewchtsverhältnis der Gesamtwirkstoffmenge von Pyrazolverbindung der Formel I und Benzoesäureverbindung der Formel II zu oberflächenaktiver Substanz S im Bereich von 1 : 10 bis 3 : 1 liegt,
wobei die Polyetherverbindung wenigstens eine Polyethergruppe der Formel III

R^{x}-[(EO)ₓ (AO)_{y}]- (III)

aufweist, worin
- EO: für -CH₂-CH₂-O- steht;
- AO: für -CHR^{a}-CR^{b}R^{c}-O- steht;
- R^{x}: für Wasserstoff, C₁-C₁₀-Alkyl, C₅-C₁₀-Cycloalkyl, Benzoyl oder C₁-C₂₀-Alkylcarbonyl steht und über das Sauerstoffatom einer EO-Gruppe oder einer AO-Gruppe gebunden ist;
- x: eine ganze Zahl bedeutet, deren Zahlenmittel im Bereich von 1 bis 150 liegt;
- y: eine ganze Zahl bedeutet, deren Zahlenmittel im Bereich von 0 bis 150 liegt, wobei das Zahlenmittel der Summe von x und y im Bereich von 5 bis 150 liegt;
- R^{a}, R^{b}: unabhängig voneinander für Wasserstoff oder Methyl stehen; und
- R^{c}: Wasserstoff, C₁-C₄-Alkyl oder Phenyl bedeutet
wobei wenigstens einer der Reste R^{a}, R^{b} und R^{c} von Wasserstoff verschieden ist, und wobei die Polyetherverbindung ausgewählt ist unter
- Ethylenoxyd-Propylenoxyd-Copolymeren,
- Polyetherverbindungen, in denen die wenigstens eine Polyethergruppe der Formel III über ein Sauerstoff-, Schwefel- oder Stickstoffatom kovalent an einen Kohlenwasserstoffrest mit 8 bis 40 Kohlenstoffatomen, der gegebenenfalls noch 1 oder 2 Carbonyloxygruppen und/oder 1, 2, 3 oder 4 OH-Gruppen aufweist, gebunden ist,
und Gemischen davon.

Zur effizienten und rentablen Durchführung einer technisierten Landwirtschaft und zur Sicherung einer gleichbleibenden Produktqualität sind Reinkulturen der landwirtschaftlich interessanten Nutzpflanzen erforderlich. Die selektive Empfindlichkeit unterschiedlicher Pflanzengruppen gegenüber bestimmten Stoffwechselinhibitoren oder anderen Zellgiften kann zur gezielten Bekämpfung von unerwünschtem Fremdpflanzenwuchs (Schadpflanzenbewuchs) auf den landwirtschaftlichen Nutzflächen genutzt werden. Hierbei ist es grundsätzlich wünschenswert, sowohl die absolute Wirksamkeit als auch die Spezifität der eingesetzten Wirkstoffe (Herbizide) gegen Schadpflanzen zu steigern.

Eine Erhöhung der Spezifität sowie in gewissen Grenzen der absoluten Wirksamkeit kann durch Verwendung von Kombinationen mehrerer spezifischer Wirkstoffe, die an unterschiedlichen Punkten des Stoffwechsels der Zielpflanzen angreifen, erreicht werden. Man spricht hierbei von Synergismus (gelegentlich auch von überadditiven Effekte), wenn die Wirkung der Kombination deutlich über der Summe der Einzelwirkungen liegt.

Eine Steigerung der absoluten Wirksamkeit von Pflanzenschutzwirkstoffen kann durch verschiedene Arten von Begleit- und Hilfssubstanzen erreicht werden, die auf unterschiedliche Weise die gewünschte Wirkung verstärken können. Weitere Zusatzstoffe können verwendet werden, um die Handhabung zu vereinfachen, die Lagerfähigkeit zu erhöhen oder anderweitige Produkteigenschaften zu verbessern.

Eine wichtige Rolle bei der Formulierung von herbiziden Wirkstoffen spielen sogenannte "Adjuvantien". Hierunter versteht man Hilfsstoffe, welche die Aktivität eines Wirkstoffs und/oder seine Selektivität gegenüber der Schadpflanze erhöhen, die jedoch für sich genommen keine oder nur eine sehr geringe Wirkung bezüglich der zu bekämpfenden Schadpflanze aufweisen. Die Wirkung von Adjuvantien für Herbizide beruht vielfach auf ihrer Grenzflächenaktivität, die den Kontakt der Applikationsform des Wirkstoffs, i. d. R. eine wässrige wirkstoffhaltige Spritzbrühe, mit der Pflanzenoberfläche verbessert sowie durch Verringerung der Oberflächenspannung das Eindringen der Applikationsform und damit des Wirkstoffs in das Erdreich verbessert. Ob eine bestimmte oberflächenaktive Substanz als Adjuvans wirkt, d. h. durch sie eine Wirkungs- oder Selektivitätssteigerung erreicht wird, hängt häufig von der Art des Wirkstoffs ab.

Adjuvantien werden in der Regel erst unmittelbar vor dem Ausbringen des Wirkstoffs der Applikationsform, z. B. der Spritzbrühe, zugesetzt. Sie können jedoch grundsätzlich auch Bestandteil einer Wirkstoffformulierung sein, was aus Gründen der Handhabung und Sicherheit der Anwendung zu bevorzugen ist. Viele Wirkstoffe sind jedoch mit den gängigen oberflächenaktiven Substanzen insbesondere bei längerer Lagerung inkompatibel. Als Inkompatibilität wird hierbei jede chemisch oder physikochemisch verursachte Wirkungsminderung oder Minderung der praktischen Anwendbarkeit bezeichnet, wie sie entweder durch direkte chemische Reaktion von Wirk- und Hilfsstoffen oder durch Verringerung der Verfügbarkeit der Wirkstoffe im Gemisch, z. B. durch Bildung von unter Anwendungsbedingungen schwerlöslichen Präzipitaten oder durch Entmischung der Formulierung, eintreten kann. Eine Inkompatibilität kann sich auch mit den anderen Bestandteilen der Formulierung ergeben. Daher ist es regelmäßig erforderlich, die Adjuvantien und den zu formulierenden Wirkstoff sowie die weiteren Bestandteile der Formulierung aufeinander abzustimmen.

Aus der WO 99/63823 ist bekannt, die Wirkung von 4-Benzoyl-substituierten Pyrazolverbindungen durch Zusatz größerer Mengen an Stickstoff-haltigen Düngemitteln und Adjuvantien zu verbessern. Bei der Applikation werden daher große Düngemittelmengen ausgebracht, was zu Selektivitätsstörungen führen kann.

Aus der WO 00/53014 ist bekannt, die Wirkung von herbiziden 4-Benzoyl-substituierten Pyrazolverbindungen durch ein Adjuvans zu steigern, das ein Gemisch aus einer Fettsäure, einem Phosphorsäure- oder Schwefelsäure-Halbester eines monohydroxyfunktionellen Polyalkylethers und ein C₁-C₅-Alkyl-C₁₀-C₂₀-alkanoat umfasst. Bei der Einarbeitung dieses Adjuvans in wässrige Konzentrat-Formulierungen der 4-Benzoyl-substituierten Pyrazolverbindungen können Homogenitäts-Probleme auftreten. Auch kann es zu einer Ausfällung des Wirkstoffs in der beim Verdünnen erhaltenen Spritzbrühe kommen. Aus diesem Grund werden derartige Adjuvantien erst kurz vor dem Ausbringen der wässrigen Spritzbrühe zugesetzt (sog. Tankmix-Verfahren).

WO 99/65314 beschreibt unter anderem synergistisch wirksame Herbizidmischungen, umfassend eine herbizid wirksame 4-Benzoyl-substituierte Pyrazolverbindung, beispielsweise eine der eingangs definierten Verbindungen I und einen Synergisten, z. B. ein Herbizid aus der Gruppe der Benzoesäureverbindungen, z. B. eine der eingangs definierten Verbindungen der Formel II. Die herbizid wirkenden Benzoesäureverbindungen führen jedoch häufig auch zu einer Schädigung von Nutzpflanzen. Das Einarbeiten von Adjuvantien in wasserlösliche Konzentrat-Formulierungen von Wirkstoffmischungen, die 4-Benzoyl-substituierte Pyrazolverbindungen der Formel I und Benzoesäureverbindungen der Formel II enthalten, ist häufig mit Problemen verbunden. Insbesondere kommt es bei Einsatz größerer Mengen an Adjuvantien und/oder höheren Konzentration an Benzoesäureverbindung häufig zu Inhomogenitäten oder zur Abscheidung von Feststoffen.

Aufgabe der vorliegenden Erfindung war es daher, eine wässrige, homogene Formulierung bereitzustellen, welche wenigstens eine Verbindungen der Formel I zusammen mit wenigstens einer Verbindungen der Formel II und eine größere Menge eines Adjuvans enthält und dabei lagerstabil ist. Die Formulierung sollte sich zudem problemlos mit Wasser verdünnen lassen.

Es wurde nun überraschenderweise gefunden, dass diese und weitere Aufgaben durch Verwendung der im Folgenden beschriebenen nichtionischen oberflächenaktiven Substanzen S gelöst werden.

Gegenstand der vorliegenden Erfindung sind daher wässrige Wirkstoffkonzentrate, enthaltend in gelöster Form:
a) mindestens eine 4-Benzoyl-substituierte Pyrazolverbindung der Formel I, wie eingangs definiert;
b) mindestens eine Benzoesäureverbindung der Formel II, wie eingangs definiert; und
c) mindestens eine nichtionische oberflächenaktive Substanz S, ausgewählt unter Polyetherverbindungen, die von Ethylenoxid abgeleitete Wiederholungseinheiten aufweisen, Alkylpolyglykosiden und Gemischen davon wie oben definiert.

Bei den erfindungsgemäßen Wirkstoffkonzentraten handelt es sich um homogene wässrige Lösungen der Wirkstoffe der Formeln I und II. Die Konzentrate sind lagerstabil und zeigen auch nach längerer Lagerung keine Tendenz zur Abscheidung von Feststoffen, auch wenn sie große Mengen an oberflächenaktiver Substanz S enthalten. Die Wirkstoffkonzentrate sind problemlos in ihrer Handhabung und lassen sich mit Wasser verdünnen, ohne dass ein Abscheiden von Wirkstoffen auftritt. Zudem wurde gefunden, dass durch Einsatz dieser nichtionischen oberflächenaktiven Substanzen S nicht nur die herbizide Wirkung der Wirkstoffmischung über die bereits bekannte Synergie der Wirkstoffe I und II hinaus erhöht werden kann sondern auch dass die schädigende Wirkung der Wirkstoffe der Formel II auf die landwirtschaftlichen Nutzpflanzen herabgesetzt wird.

Hier und im Folgenden bedeuten Alkyl und die Alkylteile in Alkylcarbonyl, Alkoxy, Alkylthio und Alkylphenyl, lineare oder verzweigte, gesättigte Kohlenwasserstoffreste. Alkenyl steht dementsprechend für lineare oder verzweigte Kohlenwasserstoffreste, die einfach ungesättigt sind. Halogenalkyl sowie die Halogenalkylteile in Halogenalkoxy stehen für lineare oder verzweigte Alkylreste, worin 1 oder mehrere, z. B. 1, 2, 3, 4, 5 oder auch alle Wasserstoffatome durch Halogen, insbesondere durch Chlor oder Fluor ersetzt sind. Phenylalkyl steht für einen Phenylrest, der mit dem Rest des Moleküls über eine Alkylgruppe gebunden ist. Cycloalkyl steht für cyclische, gesättigte Kohlenwasserstoffreste. Das Präfix Cₙ-Cₘ gibt jeweils die Anzahl möglicher Kohlenstoffatome an.

Beispiele sind Alkyl sind C₁-C₄-Alkyl wie Methyl, Ethyl, Propyl, 1-Methylethyl, Butyl, 1-Methyl-propyl, 2-Methylpropyl und 1,1-Dimethylethyl, weiterhin C₁-C₆-Alkyl, das neben den für C₁-C₄-Alkyl genannten Resten auch Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl zählen, sowie längerkettige Alkylreste wie n-Heptyl, n-Octyl, n-Nonyl, Isononyl, 2-Ethylhexyl, n-Decyl, Isodecyl, 2-Propylheptyl, Dodecyl, Tridecyl, Isotridecyl, Pentadecyl, Lauryl, Myristyl, Palmityl, Stearyl, Behenyl und dergleichen.

Alkylcarbonyl steht für einen über eine Carbonylgruppe gebundenen Alkylrest, wie zuvor genannt.

Alkoxy steht für einen über Sauerstoff gebunden Alkylrest, wie zuvor definiert, insbesondere für C₁-C₄-Alkoxy wie Methoxy, Ethoxy, Propoxy, 1-Methylethoxy, Butoxy, 1-Methyl-propoxy, 2-Methylpropoxy und 1,1-Dimethylethoxy.

Haloalkyl steht für einen Alkylrest, wie vorstehend definiert, worin ein oder mehrere, z. B. 1, 2, 3, 4 oder 5 oder alle Wasserstoffatome durch Halogen, insbesondere durch Fluor oder Chlor ersetzt sind. Beispiele sind Fluormethyl, Chlormethyl, Trifluormethyl, Difluorethyl, 2,2,2-Trifluorethyl, Pentafluorethyl 2-Fluor-1-methylethyl, 2,2,2-Trifluor-1-methylethyl, etc.

Cycloalkyl steht für einen cyclischen, gesättigten Kohlenwasserstoff-Rest wie beispielsweise Cyclopentyl, Cyclohexyl, Cycloheptyl.

Phenylalkyl steht für einen über eine Alkylgruppe gebundenen Phenylrest, wie beispielsweise Benzyl, 1- oder 2-Phenylethyl.

5-gliedrige heterocyclische Reste sind gesättigte, teilgesättigte oder aromatische Cyclen, die 5-Ringatome (Ringglieder aufweisen) und die neben den Kohlenstoffatomen als Ringglieder ein oder mehrere, z. B. 1, 2, 3 oder 4 Heteroatome, insbesondere 1 oder 2 Heteroatome als Ringglieder aufweisen, wobei die Heteroatome vorzugsweise unter O, S und N ausgewählt sind. Beispiele hierfür sind 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, Pyrrol-1-yl, Pyrazol-1-yl, Imidazol-1-yl, 1,2,3-Triazol-1-yl und 1,2,4-Triazol-1-yl.

Gemäß einer ersten Ausführungsform umfasst die Substanz S eine Polyetherverbindung, welche von Ethylenoxid abgeleitete Wiederholungseinheiten, also Wiederholungseinheiten der Formel CH₂CH₂O, und gegebenenfalls weitere von C₃-C₈-Alkylenoxiden und/oder Styroloxid abgeleitete Wiederholungseinheiten aufweist, oder ein Gemisch davon mit Alkylpolyglykosiden. In dieser Ausführungsform macht die Polyetherverbindung insbesondere wenigstens 80 Gew.-%, besonders bevorzugt wenigstens 90 Gew.-% oder die Gesamtmenge der Substanz S aus.

Derartige Polyetherverbindungen weisen typischerweise wenigstens eine, z. B. 1, 2, 3 oder 4 Polyethergruppen auf, welche neben den von Ethylenoxid abgeleitete Wiederholungseinheiten gegebenenfalls weitere, in der Regel von C₃-C₈-Alkylenoxiden und/oder Styroloxyd abgeleitete Wiederholungseinheit aufweisen. Die Polyethergruppen werden im Folgenden auch als Makrogolteil bezeichnet. In den Polyetherverbindungen sind die Polyethergruppen in der Regel kovalent an einen organischen Rest (Basisteil) gebunden oder über ein Ethersauerstoffatom zu einem Makromolekül verknüpft.

Die kovalente Anbindung des/der Makrogolteils(teile) an den Basisteil erfolgt in der Regel über ein Sauerstoff-, Schwefel- oder Stickstoffatom, vorzugsweise über ein Sauerstoffatom. Bei dem Basisteil handelt es sich typischerweise um einen organischen Rest mit in der Regel 4 bis 40, häufig 6 bis 30 und insbesondere 10 bis 22 Kohlenstoffatomen, wobei das Basisteil gegebenenfalls noch ein oder mehrere funktionelle Gruppen, z. B. 1 oder 2 Carbonyloxygruppen (C(=O)-O-Gruppen) und/oder 1, 2, 3 oder 4 OH-Gruppen und/oder 1, 2, 3, 4, 5 oder 6 Stickstoffatome aufweisen kann. Beispiele für als Basisteil geeignete Reste sind C₈-C₃₀-Alkyl, C₈-C₃₀-Alkenyl, C₄-C₃₀-Alkandiyl, C₈-C₃₀-Alkantriyl, C₅-C₁₀-Cycloalkyl, C₅-C₁₀-Cycloalkandiyl, α,α'-[Bisphenyl-C₁-C₄-alkan]diyl, α,α'-[Biscyclohexyl-C₁-C₄-alkan]diyl, Mono- und Di-C₄-C₂₀-Alkylphenyl, insbesondere Butylphenyl, 4-tert.-Butylphenyl, Hexylphenyl, Octylphenyl, Nonylphenyl, Dodecylphenyl, Tridecylphenyl, C₈-C₂₀-Alkylcarbonyl, Benzoyl, C₁-C₂₀-Alkylbenzoyl, Naphthyl, das gegebenenfalls 1, 2 oder 3 C₁-C₁₀-Alkylgruppen aufweisen kann, Mono-, Di- und Tristyrylphenyl, weiterhin von Sorbitanestern, von Alkylpolyglykosiden, von Mono- oder Diglyceriden sowie von (Oligo)alkyleniminen abgeleitete Reste.

Unter Alkyl(poly)glykosiden bzw. Alkylpolyglucosiden versteht man Verbindungen, die einen oder mehrere, insbesondere einen Alkylrest, insbesondere einen C₆-C₂₂-Alkylrest aufweisen, der über ein Sauerstoffatom an einen Mono- oder Oligosaccharid-Rest, z. B. an einen Mono-, Di oder Trisaccharid-Rest gebundenen ist. Die Saccharid-Einheiten sind dabei typischerweise von Glucose abgeleitet. Bevorzugte Alkyl(poly)glykoside sind solche, die im Mittel 1 bis 2 Glucoseeinheiten aufweisen. In der Regel handelt es sich hierbei um Gemische. In Polyetherverbindungen, welche einen von Alkyl(poly)-glykosiden abgeleiteten Basisteil aufweisen, ersetzt der wenigstens eine Makrogolteil wenigstens eine der nicht veresterten Hydroxylgruppen des Mono- bzw. Oligosaccharid-Rests.

Unter Sorbitanestern versteht man Ester, insbesondere Mono- oder Diester von Sorbitol mit gesättigten oder ungesättigten, aliphatischen Carbonsäuren, insbesondere gesättigten oder ungesättigten Fettsäuren mit 8 bis 22 C-Atomen. In Polyetherverbindungen, welche einen von Sorbitanestern abgeleiteten Basisteil aufweisen, ersetzt der wenigstens eine Makrogolteil wenigstens eine der nicht veresterten Hydroxylgruppen des Sorbitans.

Unter Mono- und Diglyceriden versteht man Mono- bzw. Diester von Glycerin bzw. der en Gemische mit gesättigten oder ungesättigten, aliphatischen Carbonsäuren, insbesondere gesättigten oder ungesättigten Fettsäuren mit 8 bis 22 C-Atomen. In Polyetherverbindungen, welche einen von Mono- oder Diglyceriden abgeleiteten Basisteil aufweisen, ersetzt der wenigstens eine Makrogolteil wenigstens eine der nicht veresterten Hydroxylgruppen des Glycerins.

Unter von (Oligo)alkyleniminen abgeleitete Reste versteht man solche Reste, die von Alkylendiaminen oder oligomeren Iminoalkylenaminen wie Mono-, Di-, Tri- und Tetraethylenimin oder Mono-, Bis-, Tris- oder Tetrakis-(3-Aminopropyl)ethylendiamin abgeleitet sind. In Polyetherverbindungen, welche einen von (Oligo)alkylenniminen abgeleiteten Basisteil aufweisen, ersetzt der wenigstens eine Makrogolteil wenigstens ein NH-Wasserstoffatom des (Oligo)alkylenimins.

Der Anteil der EO-Wiederholungseinheiten am Gesamtgewicht der Polyetherverbindungen liegt typischerweise im Bereich von 10 bis 90 Gew.-% und insbesondere im Bereich von 30 bis 85 Gew.-%.

In der Regel weisen die Polyetherverbindungen einen HLB-Wert nach Griffin von 1,5 bis 19,5, bevorzugt von 1,5 bis 14,0, besonders bevorzugt 2 bis 10, ganz besonders bevorzugt von 3 bis 7 auf. Die genannten Zahlen beziehen sich hierbei stets auf einen Mittelwert. Der "HLB-Wert nach Griffin" bedeutet hierbei das Verhältnis der hydrophilen und hydrophoben Teile des Moleküls, ausgedrückt als das Zwanzigfache des Anteils des Ethylenoxidteils am Molekulargewicht des gesamten Moleküls.

Polyetherverbindungen, deren Polyethergruppen neben den von Ethylenoxid abgeleiteten Wiederholungseinheiten auch andere, d. h. von C₃-C₅-Alkylenoxiden und/oder Styroloxyd abgeleitete Wiederholungseinheiten aufweisen, haben in der Regel einen modifizierten HLB-Wert im Bereich von 5 bis 19,5, bevorzugt von 5 bis 16, besonders bevorzugt von 7 bis 14, ganz besonders bevorzugt von 10 bis 14; die genannten Zahlen beziehen sich hierbei stets auf einen Mittelwert. Der "modifizierte HLB-Wert" bedeutet hierbei das Verhältnis der hydrophilen und hydrophoben Teile des Moleküls, zur Berücksichtigung der unterschiedlichen Hydrophilie von Ethylenoxid-Einheiten und sonstigen Wiederholungseinheiten in der Polyetherkette, ausgedrückt als das Zwanzigfache des Anteils des Ethylenoxidteils am Molekulargewicht des gesamten Moleküls plus das Zehnfache des Anteils der sonstigen Wiederholungseinheiten am Molekulargewicht des gesamten Moleküls.

Das Molekulargewicht der Polyetherverbindungen kann über weite Bereich variieren und liegt typischerweise im Bereich von 200 bis 10000 Dalton und insbesondere im Bereich von 300 bis 5000 Dalton (jeweils Zahlenmittel), soweit nichts anderes angegeben ist. Vorzugsweise liegt der Quotient aus Massen- und Zahlenmittel des Molekulargewichts im Bereich von 0,9 und 1,6, bevorzugt im Bereich von 1,0 und 1,4 und besonders bevorzugt im Bereich von 1,1 und 1,3.

Wie bereits oben erläutert, lassen sich die Polyethergruppen in den Polyetherverbindungen S in der Regel durch die allgemeine Formel III

R^{x}-[(EO)ₓ (AO)_{y}]- (III)

beschreiben, worin
- EO: für -CH₂-CH₂-O- steht;
- AO: für -CHR^{a}-CR^{b}R^{c}-O- steht, wobei die Anordnung von EO-Einheiten und AO-Einheiten innerhalb der Kette beliebig, einschließlich einer statistischen Anordnung oder einer Blockanordnung sein kann;
- R^{x}: für Wasserstoff, C₁-C₁₀-Alkyl, C₅-C₁₀-Cycloalkyl, Benzyl oder C₁-C₂₀-Alkylcarbonyl steht und über das Sauerstoffatom einer EO-Gruppe oder einer AO-Gruppe gebunden ist;
- x: eine ganze Zahl bedeutet, deren Zahlenmittel im Bereich von 1 bis 150 liegt, insbesondere von 2 bis 80 und besonders bevorzugt von 3 bis 40 liegt;
- y: eine ganze Zahl bedeutet, deren Zahlenmittel im Bereich von 0 bis 150 liegt, insbesondere von 0 bis 50 und speziell von 0 bis 30 liegt, wobei das Zahlenmittel der Summe von x und y in der Regel im Bereich von 2 bis 150 liegt, insbesondere im Bereich von 3 bis 80 und besonders bevorzugt im Bereich von 5 bis 40 liegt;
- R^{a}, R^{b}: unabhängig voneinander für Wasserstoff oder Methyl stehen und insbesondere Wasserstoff bedeuten; und
- R^{c}: Wasserstoff, C₁-C₄-Alkyl, speziell Methyl oder Phenyl bedeutet,
wobei wenigstens einer der Reste R^{a}, R^{b} und R^{c} von Wasserstoff verschieden ist.

In Formel III stellen AO und EO Wiederholungseinheiten (Monomereinheiten) dar, aus denen die Polyethergruppe aufgebaut ist. Wenn die Polyethergruppen der Formel III Wiederholungseinheiten AO umfassen, können die Wiederholungseinheiten EO und AO beliebig angeordnet sein, z. B. in einer Blockanordnung, worin längere Folgen von EO-Einheiten mit längeren Folgen von AO-Einheiten verknüpft sind, oder in einer regellosen, d. h. statistischen Anordnung oder in Mischformen aus statistischer Anordnung und Blockanordnung. Sofern die Polyethergruppen III eine Blockanordnung von AO-Blöcken und EO-Blöcken aufweisen, ist es bevorzugt, wenn die Polyethergruppe aus 2 oder 3 und insbesondere aus 2 Blöcken besteht. Die Indices x und y geben dabei die Anzahl der jeweiligen Wiederholungseinheiten innerhalb der Polyethergruppe an. Da es sich bei den Polyetherverbindungen in der Regel nicht um molekular einheitliche Verbindungen sondern um Gemische von Verbindungen mit unterschiedlichen Polyetherketten handelt, die sich typischerweise in der Anzahl der jeweiligen Wiederholungseinheiten unterscheiden, handelt es sich bei den Angaben x und y typischerweise um Mittelwerte (Zahlenmittel), jeweils bezogen auf die Gesamtmenge an Wiederholungseinheiten EO bzw. AO in der Polyetherverbindung.

Als Gruppe AO sind beispielsweise Reste zu nennen, die von Propylenoxid (PO; R^{a} = R^{b} = Wasserstoff und R^{c} = Methyl), Butylenoxid (BO; R^{a} = R^{b} = Wasserstoff und R^{e} = Ethyl), Isobutylenoxid (IBO; R^{a} = Wasserstoff und R^{b} = R^{c} = Methyl), Pentylenoxid (PPO; R^{a} = R^{b} = Wasserstoff und R^{c} = Propyl), Hexylenoxid (HO; R^{a} = R^{b} = Wasserstoff und R^{c} = Butyl) und Styroloxid (StO; R^{a} = R^{b} = Wasserstoff und R^{c} = Phenyl) abgeleitet sind. Sofern die Polyethergruppe Reste AO aufweist, sind diese vorzugsweise von Propylenoxid abgeleitet.

Unter den Polyetherverbindungen sind solche mit einer oder mehreren Gruppen der Formel III bevorzugt, worin R^{x} für Wasserstoff oder C₁-C₁₀-Alkyl steht, insbesondere für Wasserstoff oder C₁-C₄-Alkyl, speziell Methyl. In einer bevorzugten Ausführungsform der Erfindung sind die Polyethergruppen Endgruppen-modifiziert, d. h. R^{x} steht für einen von Wasserstoff verschiedenen Rest. Vorzugsweise steht dann R^{x} für C₁-C₁₀-Alkyl, insbesondere für C₁-C₄-Alkyl und speziell für Methyl. Als Substanz S sind insbesondere auch Polyetherverbindungen geeignet, worin R^{x} für C₁-C₂₀-Alkylcarbonyl steht. Gemäß einer besonders bevorzugten Ausführungsform sind die Polyetherverbindungen nicht endgruppenmodifiziert, d. h. R^{x} ist Wasserstoff.

In einer bevorzugten Ausführungsform ist die Polyetherverbindung ausgewählt unter Ethylenoxyd-Propylenoxyd-Copolymeren (nachfolgend EO/PO-Copolymere genannt). Hierunter versteht man Polyetherverbindungen, die überwiegend, d. h. zu wenigstens 90 Gew.-% aus Wiederholungseinheiten EO und PO (= CH₂-CH(CH₃)-O) aufgebaut sind. Formal handelt es sich hierbei um Verbindungen, worin zwei Polyethergruppen der Formel III, worin y^{≠} 0 ist und AO für CH₂-CH(CH₃)O steht, über ein Ethersauerstoffatom oder über eine C₄-C₁₀-Alkandiylgruppe miteinander verknüpft sind. Hierunter sind Ethylenoxid-Propylenoxid-Blockcopolymere bevorzugt, wobei die Anzahl der PO-Blöcke und der EO-Blöcke vorzugsweise 2 oder insbesondere 3 beträgt. Insbesondere bevorzugt sind Triblockcopolymere der folgenden Formeln

R^{x}[EOₓ₁][PO_{y3}][EOₓ₂]OR^{x'}

R^{x}[EOₓ₁][PO_{y1}]Y-A-Y[PO_{y2}][EOₓ₂]R^{x'}

R^{x}[PO_{y1}][EOₓ₃][PO_{y2}]OR^{x'}

Hierbei wird die Einheit [PO_{y1}]A[PO_{y2}] als ein PO-Block betrachtet. In den Formeln haben R^{x}, EO, PO, x und y die zuvor genannten Bedeutungen und R^{x'} hat eine der für R^{x} angegebenen Bedeutungen. Die Indices x1 und x2 weisen unabhängig voneinander einen der für x angegebenen Werte auf. Die Indices y1 und y2 sind von 0 verschieden und weisen im Übrigen unabhängig voneinander einen der für y angegebenen Werte auf. Der Index y3 steht typischerweise für einen Wert von 2 bis 160, insbesondere für einen Wert von 4 bis 100 und speziell für 10 bis 80. Der Index x3 steht typischerweise für einen Wert von 4 bis 200, insbesondere für einen Wert von 10 bis 100 und speziell für 10 bis 80. A steht für C₄-C₁₀-Alkandiyl oder C₅-C₁₀-Cycloalkandiyl. Y steht für Sauerstoff oder für einen Rest NR, worin R für Wasserstoff, C₁-C₄-Alkyl oder eine Gruppe der Formel III steht. R^{x} und R^{x'} stehen insbesondere für Wasserstoff oder C₁-C₁₀-Alkyl. Das zahlenmittlere Molekulargewicht der EO/PO-Copolymere liegt vorzugsweise im Bereich von 300 bis 10000 Dalton, insbesondere im Bereich von 500 bis 5000 Dalton. Der Anteil der EO-Wiederholungseinheiten liegt typischerweise im Bereich von 10 bis 90 Gew.-%, insbesondere im Bereich von 20 bis 80 Gew.-% und der Anteil der PO-Wiederholungseinheiten im Bereich von 10 bis 90 Gew.-%, insbesondere im Bereich von 20 bis 80 Gew.-%, jeweils bezogen auf die Gesamtgewicht des EO/PO-Copolymers.

Gemäß einer weiteren bevorzugten Ausführungsform der Erfindung ist die Polyetherverbindung ausgewählt unter Polyetherverbindungen, die wenigstens eine, beispielsweise 1, 2, 3 oder 4, insbesondere 1 oder 2, und speziell eine Polyethergruppe der Formel III aufweisen, welche über ein Sauerstoff-, Schwefel- oder Stickstoffatom kovalent an einen Kohlenwasserstoffrest mit 8 bis 40 Kohlenstoffatomen, insbesondere 10 bis 30 C-Atomen, der gegebenenfalls noch 1 oder 2 Carbonyloxygruppen und/oder 1, 2, 3 oder 4 OH-Gruppen aufweist, gebunden ist (sind).

Bevorzugte Polyetherverbindungen dieser Ausführungsform sind:
- Polyethoxylate und Poly(ethoxylat-co-propoxylat)e von C₈-C₂₂-Alkanolen, insbesondere C₁₀-C₁₈-Alkanolen. Hierunter versteht man Verbindungen, die eine Gruppe der Formel III aufweisen, welche über ein Sauerstoffatom an einen C₈-C₂₂-Alkyl-Rest gebunden ist, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Fettsäuren. Hierunter versteht man Verbindungen, die eine Gruppe der Formel III aufweisen, welche über ein Sauerstoffatom an einen Fettsäure-Rest, in der Regel ein C₈-C₂₂-Alkylcarbonyl-Rest oder ein C₈-C₂₂-Alkenylcarbonyl-Rest gebunden ist,
wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Fettaminen. Hierunter versteht man Verbindungen, die eine oder zwei Gruppen der Formel III aufweisen, welche über ein Stickstoffatom an einen von einem Fettamin abgeleiteten Kohlenwasserstoffrest, in der Regel ein C₈-C₂₂-Alkyl-Rest gebunden sind, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Mono- und Diglyceriden aliphatischer C₈-C₂₂-Monocarbonsäuren. Hierunter versteht man Verbindungen, die eine oder zwei Gruppen der Formel III aufweisen, welche über ein Sauerstoffatom an einen von einem Mono- oder Diglycerid einer gesättigten oder ungesättigten, aliphatischen C₈-C₂₂-Monocarbonsäure abgeleiteten Rest gebunden sind, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Sorbitanestern gesättigter oder ungesättigter aliphatischer C₈-C₂₂-Monocarbonsäuren. Hierunter versteht man Verbindungen, die eine oder zwei Gruppen der Formel III aufweisen, welche über ein Sauerstoffatom an einen von einem Sorbitanmono- oder -diester einer aliphatischer C₈-C₂₂-Monocarbonsäure abgeleiteten Rest gebunden sind, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Alkylphenolen. Hierunter versteht man Verbindungen, die eine Gruppe der Formel III aufweisen, welche über ein Sauerstoffatom an einen Alkylphenyl-Rest, insbesondere an einen Mono- oder Di-C₄-C₂₀-alkylphenyl-Rest gebunden ist, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Mono-, Di- und Tristyrylphenolen. Hierunter versteht man Verbindungen, die eine Gruppe der Formel III aufweisen, welche über ein Sauerstoffatom an einen Mono-, Di- oder Tristyrylphenyl-Rest gebunden ist, wobei die Gruppe der Formel III entweder ausschließlich Wiederholungseinheiten EO oder Wiederholungseinheiten EO und PO aufweist;
- Polyethoxylate und Poly(ethoxylat-co-propoxylat)e von Alkyl(poly)glykosiden und Gemischen davon. Hierunter versteht man Verbindungen, die eine oder mehrere, z. B. 1, 2, 3 oder 4 Gruppen der Formel III aufweisen, welche über ein Sauerstoffatom an einen von einem Alkyl(poly)glykosid abgeleiteten Rest gebunden sind, wobei die Gruppe der Formel III entweder ausschließlich Wiederholungseinheiten EO oder Wiederholungseinheiten EO und PO aufweist.

Neben den vorgenannten Polyetherverbindungen, sind auch Polyethoxylate und Poly-(ethoxylat-co-propoxylat)e von Oligo- oder Polyalkyleniminen, insbesondere solche von Verbindungen der Formel NH₂-(A-NH)ₖ-A'-NH₂, worin A und A' unabhängig voneinander für Ethan-1,2-diyl oder Propan-1,3-diyl stehen, und k im Bereich von 1 bis 100 liegt, geeignet.

Die vorgenannten Polyethoxylate und Poly(ethoxylat-co-propoxylat)e können Endgruppen-verschlossen sein, d. h. der Rest R^{x} ist von Wasserstoff verschieden und steht insbesondere für C₁-C₁₀-Alkyl, vorzugsweise C₁-C₄-Alkyl und speziell für Methyl. Ebenfalls bevorzugt sind solche der vorgenannten Polyethoxylate und Poly(ethoxylat-co-propoxylat)e, worin der Rest R^{x} für Wasserstoff steht.

Das zahlenmittlere Molekulargewicht der vorgenannten Polyethoxylate und Poly(ethoxylat-co-propoxylat)e liegt vorzugsweise im Bereich von 200 bis 5000 Dalton, insbesondere im Bereich von 300 bis 3000 Dalton. Der Anteil der EO-Wiederholungseinheiten liegt typischerweise im Bereich von 7 bis 98 Gew.-%, insbesondere im Bereich von 10 bis 80 Gew.-%, und speziell 15 bis 70 Gew.-%, bezogen auf das Gesamtgewicht der Polyethoxylate bzw. Poly(ethoxylat-co-propoxylat)e. Bei den Poly-(ethoxylat-co-propoxylat)en können die PO und EO-Wiederholungseinheiten statistisch oder blockweise angeordnet sein, wobei letzteres bevorzugt ist. Insbesondere weisen die Poly(ethoxylat-co-propoxylat)e einen Block aus EO-Wiederholungseinheiten auf, welcher an den Basisteil der Verbindung gebunden ist, und einen Block aus PO-Wiederholungseinheiten, welcher den Rest R^{x} trägt. Hierunter sind insbesondere solche Polyethoxylate mit einem mittleren Ethoxylierungsgrad (entsprechend dem Zahlenmittel von x) im Bereich von 3 bis 50, insbesondere 4 bis 30 und speziell 5 bis 20 bevorzugt, Unter den Poly(ethoxylat-co-propoxylat)en sind solche mit einem mittleren Ethoxilierungsgrad im Bereich von 2 bis 49, vorzugsweise im Bereich von 3 bis 29 und speziell im Bereich von 4 bis 19 und einem mittleren Propoxylierungsgrad (entsprechend dem Zahlenmittel von y) im Bereich von 1 bis 48, insbesondere im Bereich von 1 bis 27 und speziell im Bereich von 1 bis 16 bevorzugt, wobei der Gesamt-Alkoxylierungsgrad (entsprechend dem Zahlenmittel der Summe x+y) vorzugsweise im Bereich von 3 bis 50, speziell 4 bis 30 und ganz besonders bevorzugt im Bereich von 5 bis 20 liegt.

Gemäß einer besonders bevorzugten Ausführungsform handelt es sich bei der Polyetherverbindung um ein polyalkoxyliertes C₈-C₃₀-Alkanol. Derartige Verbindungen können durch die allgemeine Formel IV

R¹¹-O-[EOₓ;AO_{y}]-R^{x} (IV)

beschrieben werden, worin EO, AO, x, y, und R^{x} die zuvor genannten Bedeutungen aufweisen und R¹¹ für einen linearen oder verzweigten Alkylrest mit 8 bis 30 C-Atomen, insbesondere mit 8 bis 22 C-Atomen und speziell mit 10 bis 18 C-Atome steht. Bevorzugte lineare Alkylreste R¹¹ leiten sich von Alkanolen mit 8 bis 22 C-Atomen, insbesondere mit 10 bis 18 C-Atomen ab. Besonders bevorzugte Alkylreste R¹¹ sind wenigstens einfach verzweigt und weisen 8 bis 22 C-Atome, insbesondere 10 bis 18 C-Atome auf. Beispiele für R¹¹ sind lineare Reste wie n-Octyl, n-Decyl, n-Dodecyl, n-Tridecyl, n-Tetradecyl, n-Pentadecyl, n-Hexadecyl, n-Octadecyl, und verzweigte Reste wie iso-Nonyl, iso-Undecyl, iso-Tridecyl, iso-Pentadecyl, 2-Ethylhexyl und 2-Propylheptyl. Hierbei ist zu beachten, dass in den Verbindungen IV die Reste R¹¹ auch ein Gemisch unterschiedlicher Reste mit vorzugsweise gleicher oder ähnlicher C-Zahl und unterschiedlichen Verzweigungsgraden sein können, wie sie bei der technischen Herstellung der den Verbindungen IV zugrundeliegenden Alkanolen anfallen.

Unter den Polyetherverbindungen der Formel IV sind solche bevorzugt, worin AO, sofern vorhanden, für CH₂CH(CH₃) steht. Hierunter sind insbesondere solche Verbindungen bevorzugt, worin x eine Zahl bedeutet, deren Zahlenmittel im Bereich von 2 bis 49, vorzugsweise im Bereich von 3 bis 39 und speziell im Bereich von 4 bis 29 liegt, y eine Zahl bedeutet, deren Zahlenmittel im Bereich von 1 bis 48, insbesondere im Bereich von 1 bis 37 und speziell im Bereich von 1 bis 26 liegt, und das Zahlenmittel der Summe x+y im Bereich von 3 bis 50, speziell im Bereich von 4 bis 40 liegt. In besonders bevorzugten Poly(ethoxylat-co-propoxylat)en der Formel IV sind die EO-Einheiten und die PO-Einheit in Form zweier Blöcke angeordnet. Die Polyetherverbindungen IV können Endgruppen-verschlossen sein, d. h. R^{x} ist von Wasserstoff verschieden und steht vorzugsweise für C₁-C₁₀-Alkyl, insbesondere für C₁-C₄-Alkyl und speziell für Methyl. Insbesondere steht R^{x} für Wasserstoff.

Gemäß einer weiteren besonders bevorzugten Ausführungsform handelt es sich bei der Polyetherverbindung um ein polyalkoxyliertes Alkylphenol oder ein polyalkoxyliertes Mono-, Di- oder Tristyrylphenol. Derartige Verbindungen können durch die allgemeine Formel V

R¹²-O-[EOₓ;AO_{y}]-R^{x} (V)

beschrieben werden, worin EO, AO, x, y, und R^{x} die zuvor genannten Bedeutungen aufweisen und R¹² für einen Phenylrest steht, der einen oder zwei lineare oder verzweigte Alkylreste mit in der Regel 4 bis 20 C-Atomen, insbesondere 6 bis 16 C-Atomen oder 1, 2 oder 3 von Styrol abgeleitete Reste trägt. Beispiele für Alkylreste an Phenyl umfassen n-Butyl, tert.-Butyl, n-Hexyl, n-Octyl, n-Decyl, n-Dodecyl, n-Tetradecyl, n-Hexadecyl, n-Octadecyl, iso-Nonyl, Undecyl, Tridecyl, 2-Ethylhexyl und 2-Propylheptyl.

Unter den Polyetherverbindung der Formel V sind solche bevorzugt, worin R^{x} in Formel III für C₁-C₁₀-Alkyl steht und AO, sofern vorhanden, für CH₂CH(CH₃) steht. Hierunter sind insbesondere solche Verbindungen bevorzugt, worin x eine Zahl bedeutet, deren Zahlenmittel im Bereich von 2 bis 49, vorzugsweise im Bereich von 3 bis 29 und speziell im Bereich von 4 bis 19 liegt, y eine Zahl bedeutet, deren Zahlenmittel im Bereich von 1 bis 48, insbesondere im Bereich von 1 bis 27 und speziell im Bereich von 1 bis 16 liegt, und das Zahlenmittel der Summe x+y im Bereich von 3 bis 50, speziell 4 bis 30 und ganz besonders bevorzugt im Bereich von 5 bis 20 liegt. In besonders bevorzugten Poly(ethoxylat-co-propoxylat)en der Formel V sind die EO-Einheiten und die PO-Einheit in Form zweier Blöcke angeordnet.

In einer zweiten bevorzugten Ausführungsform umfasst die Substanz S wenigstens ein Alkylpolyglykosid. In dieser Ausführungsform ist der Anteil des Alkylpolyglykosids an der Substanz S typischerweise wenigstens 90 Gew.-%. In einer weiteren bevorzugten Ausführungsform handelt es sich es bei der Substanz S um ein Gemisch aus Alkylpolyglykosid mit wenigstens einer Polyetherverbindung, insbesondere einer Polyetherverbindung der Formeln IV oder V. Das Gewichtsverhältnis von Alkylglykosid zu Polyetherverbindung liegt dann typischerweise im Bereich von 9:1 bis 1:9, insbesondere im Bereich von 2:8 bis 8:2.

Die vorgenannten Substanzen S sind dem Fachmann bekannt und im Handel erhältlich. Typische Handelsprodukte der Formel IV werden z. B. von der Fa. BASF unter dem gemeinsamen Markennamen der "Lutensole" angeboten, wobei man je nach Basisteil Lutensole der Serien A, AO, AT, ON, AP, XP, XL, TO und FA unterscheidet. Weiterhin hinzugefügte Zahlen geben den Ethoxylierungsgrad an. So ist z. B. "Lutensol AO 8" ein C₁₃₋₁₅-Oxoalkohol mit acht EO-Einheiten. "Lutensol FA" steht für eine Reihe alkoxylierter Amine.

Weitere Beispiele für erfindungsgemäß geeignete Polyetherverbindungen sind Produkte der Fa. Akzo, z. B. die "Ethylan"-Reihe auf der Basis linearer oder verzweigter Alkohole. So ist z. B. "Ethylan SN 120" ein C₁₀₋₁₂-Alkohol mit zehn EO-Einheiten, und "Ethylan 4 S" ein C₁₂₋₁₄-Alkohol mit vier EO-Einheiten.

Weitere Beispiele für erfindungsgemäß geeignete Polyalkoxylate sind ferner die "NP"-Produkte der Fa. Akzo (vormals Fa. Witco) auf der Basis von Nonylphenolen.

Erfindungsgemäß geeignete Polyetherverbindungen sind auch so genannte "narrow range"-Produkte. Der Ausdruck "narrow range" bezieht sich hierbei auf eine engere Verteilung der Anzahl der EO-Einheiten. Hierzu gehören z. B. Produkte der "Berol"-Reihe von Akzo.

Erfindungsgemäß sind ferner Sorbitanester-Ethoxylate, z. B. "Armotan AL 69-66 POE(30) Sorbitan-monotallate", also eine mit Sorbitol veresterte und anschließend ethoxylierte ungesättigte Fettsäuren.

In einer bevorzugten Ausführungsform der Erfindung enthält das wässrige Wirkstoffkonzentrat die Komponenten a) und b) in Form ihrer gelösten Salze, insbesondere in Form ihrer gelösten Alkalimetall- oder Ammoniumsalze, bevorzugt in Form ihrer gelösten Natrium-, Kalium- oder Ammoniumsalze. Der pH-Wert des wässrigen Wirkstoffkonzentrats beträgt vorzugsweise mindestens pH 8,0 und liegt insbesondere im Bereich von pH 8,0 bis 10,0, besonders bevorzugt im Bereich von pH 8,0 bis 9,0.

Die vorliegende Erfindung betrifft insbesondere wässrige Wirkstoffkonzentrate von Verbindungen der Formel I, worin R¹ und R³ unabhängig voneinander vorzugsweise für Halogen, Methyl, Methylthio, Methylsulfinyl oder Methylsulfonyl stehen. R² steht insbesondere für einen unter Thiazol-2-yl, Thiazol-4-yl, Thiazol-5-yl, Isoxazol-3-yl, Isoxazol-4-yl, Isoxazol-5-yl, 4,5-Dihydroisoxazol-3-yl, 4,5-Dihydroisoxazol-4-yl und 4,5-Dihydrisoxazol-5-yl ausgewählten Rest, wobei die vorgenannten Reste unsubstituiert sind oder in der oben genannten Weise substituiert sein können und insbesondere unsubstituiert sind oder 1 oder 2 Methylgruppen als Substituenten tragen können. Insbesondere ist R² ausgewählt unter Isoxazol-5-yl, 3-Methyl-isoxazol-5-yl, 4,5-Dihydroisoxazol-3-yl, 5-Methyl-4,5-dihydroisoxazol-3-yl, 5-Ethyl-4,5-dihydroisoxazol-3-yl oder 4,5-Dimethyl-4,5-dihydroisoxazol-3-yl. R⁴ steht insbesondere für Wasserstoff. R⁵ für steht insbesondere für Methyl. R⁶ steht insbesondere für Wasserstoff oder Methyl. Insbesondere steht R¹ für Chlor, Methyl oder Methylsulfonyl, R² für Wasserstoff oder 4,5-Dihydro-isoxazol-3-yl, R³ für Chlor oder Methylsulfonyl, R⁴ für Wasserstoff, R⁵ für Methyl und R⁶ für Wasserstoff oder Methyl steht.

In einer besonders bevorzugten Ausführungsform der Erfindung ist R¹ Methyl, R² 4,5-Dihydro-isoxazol-3-yl, R³ Methylsulfonyl, R⁴ Wasserstoff, R⁵ Methyl und R⁶ Wasserstoff, d. h. die Komponente a) enthält 4-[2-Methyl-3-(4,5-Dihydroisoxazol-3-yl)-4-methylsulfonyl-benzoyl]-1-methyl-5-hydroxy-1 H-pyrazol (Common name: Topramezon).

Die vorliegende Erfindung betrifft insbesondere wässrige Wirkstoffkonzentrate von Verbindungen der Formel II, worin R⁷ für Wasserstoff oder Methoxy und R⁸ für Wasserstoff, Chlor oder Amino steht. Insbesondere steht R⁷ für Methoxy. In einer besonders bevorzugten Verbindung der Formel II steht R⁷ für Methoxy und R⁸ für Wasserstoff steht. In einer anderen besonders bevorzugten Verbindung der Formel II steht R⁷ für Wasserstoff und R⁸ für Amino. Es ist äußerst bevorzugt, wenn die Komponente b) 3,6-Dichlor-ortho-Anissäure (Common name: Dicamba) enthält.

In besonderem Maße bevorzugt ist es, wenn die wässrige Flüssigformulierung als Komponente a) 4-[2-Methyl-3-(4,5-Dihydroisoxazol-3-yl)-4-methylsulfonyl-benzoyl]-1-methyl-5-hydroxy-1 H-pyrazol in gelöster Form und als Komponente b) 3,6-Dichlor-ortho-Anissäure in gelöster Form enthält.

Die Konzentration an 4-Benzoyl-substituierten Pyrazolverbindungen der Formel I in dem erfindungsgemäßen Wirkstoffkonzentrat beträgt in der Regel 10 bis 100 g/l und insbesondere 25 bis 80 g/l. Die Konzentration an Benzoesäureverbindung der Formel II in dem erfindungsgemäßen Wirkstoffkonzentrat beträgt in der Regel 50 bis 250 g/l und insbesondere 80 bis 200 g/l und speziell 140 bis 160 g/l. Die Gesamtkonzentration an nicht-ionischer oberflächenaktiver Substanz S liegt in den erfindungsgemäßen wässrigen Wirkstoffkonzentraten in der Regel im Bereich von 100 bis 300 g/l, insbesondere im Bereich von 200 bis 400 g/l.

In einer besonderen Ausführungsform der Erfindung enthält die wässrige Flüssigformulierung eine herbizid wirksame 4-Benzoyl-substituierte Pyrazolverbindung der Formel I und ein herbizid wirksames Benzoesäurederivat der Formel II in einem relativen Mengenverhältnis (Gewichtsverhältnis) von 1:25 bis 2:1, bevorzugt 1:10 bis 1:1 und besonders bevorzugt 1:5 bis 1:3.

Erfindungsgemäß enthält die wässrige Flüssigformulierung ein Wirkstoffgemisch aus einer herbizid wirksamen 4-Benzoyl-substituierten Pyrazolverbindung der Formel I und einem herbizid wirksamen Benzoesäurederivat der Formel II sowie eine nicht-ionische oberflächenaktive Substanz S, wobei das Mengenverhältnis (Gewichtsverhältnis) der Gesamtmenge des Wirkstoffgemischs zu der Menge der Substanz S im Bereich von 1:10 bis 3:1, bevorzugt 1:3 bis 3:2 und besonders bevorzugt 2:3 bis 1:1 1 liegt.

Die Verbindungen, die die Substanz S umfassen, sind im Stand der Technik zumindest teilweise beschrieben.

Die erfindungsgemäßen wässrigen Wirkstoffkonzentrate können zusätzlich noch weitere Stoffe enthalten, die nicht im unmittelbaren Zusammenhang mit der Zielsetzung der Zusammensetzungen stehen, aber ihre Anwendbarkeit und/oder praktischen Eigenschaften verbessern. Beispiele hierfür sind insbesondere
- Viskositäts-Regler (Verdicker)
- Konservierungsmittel,
- Antischaummittel,
- Mittel zur Einstellung des pH-Werts,
- Frostschutzmittel.

Entsprechende Stoffe sind dem Fachmann geläufig. Die Gesamtmenge derartiger Stoffe wird in der Regel 10 Gew.-%, bezogen auf das Wirkstoffkonzentrat nicht überschreiten und liegt typischerweise im Bereich von 0.1 bis 10 Gew.-%, bezogen auf das Gesamtgewicht der Wirkstoffkonzentrats.

Zu den Viskosität verändernden Additiven (Andicker) zählen insbesondere Verbindungen, die bekanntermaßen wässrigen Formulierungen ein pseudoplastisches Fließverhalten verleihen, d. h. hohe Viskosität im Ruhezustand und niedrige Viskosität im bewegten Zustand. Geeignet sind grundsätzlich alle für diesen Zweck in wässrigen Wirkstoffkonzentraten eingesetzte Verbindungen. Zu nennen sind beispielsweise anorganische Substanzen wie Bentonite oder Attapulgite (z. B. Attaclay^{®} Firma Engelhardt), und organische Substanzen wie Polysaccharide und Heteropolysaccharide wie Xanthan Gum^{®} (Kelzan^{®} der Fa. Kelco), Rhodopol^{®} 23 (Rhone Poulenc) oder Veegut^{®} (Firma R.T. Vanderbilt) zu nennen, wobei Xanthan-Gum^{®} bevorzugt verwendet wird. Die Menge der die Viskosität verändernden Additive beträgt häufig 0,1 bis 5 Gew.-%, bezogen auf das Gesamtgewicht des Wirkstoffkonzentrats.

Als geeignete Antischaummittel kommen beispielsweise für diesen Zweck bekannte Silikonemulsionen (Silikon^{®} SRE, Firma Wacker oder Rhodorsil^{®} der Firma Rhodia), langkettige Alkohole, Fettsäuren, Entschäumer vom Typ wässriger Wachsdispersionen, feste Entschäumer (sog. Compounds), fluororganische Verbindungen und deren Gemische in Betracht. Die Menge an Antischaummittel beträgt typischerweise 0,1 bis 1 Gew.-%, bezogen auf das Gesamtgewicht des Wirkstoffkonzentrats.

Beispiele für Konservierungsmittel sind solche auf Basis von Isothiazolonen, beispielsweise Proxel^{®} der Fa. ICI oder Acticide^{®} RS der Fa. Thor Chemie oder Kation^{®} MK der Firma Rohm & Haas. Die Menge an Konservierungsmitteln beträgt, sofern vorhanden, typischerweise 0,05 bis 0,5 Gew.-%, bezogen auf das Gesamtgewicht des Wirkstoffkonzentrats.

Geeignete Frostschutzmittel sind flüssige Alkanole wie Methanol, Ethanol, Isopropanol, n-Butanol, Polyole, z. B. Ethylenglycol, Propylenglycol oder Glycerin. Die Menge an Frostschutzmitteln beträgt, sofern vorhanden, in der Regel 1 bis 10 Gew.-%, bezogen auf das Gesamtgewicht des Wirkstoffkonzentrats.

Gegebenenfalls können die erfindungsgemäßen Wirkstoffkonzentrate Mittel zur pH-Wert Regulation enthalten. Beispiele für solche mittel sind Basen, z. B. Alkalimetallhydroxide wie Kaliumhydroxid, Natriumhydroxid, Natriumcarbonat, Kaliumcarbonat oder Ammoniak aber auch Puffer, z. B. Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure. Die Menge an Mitteln zur Einstellung des pH-Werts beträgt, sofern vorhanden, in der Regel 0,01 bis 3 Gew.-%, bezogen auf das Gesamtgewicht des Wirkstoffkonzentrats.

Die erfindungsgemäßen wässrigen Wirkstoffkonzentrate können in einfacher Weise hergestellt werden, indem man die Wirkstoffe der Formeln I und II in Wasser bzw. einem wässrigen Medium löst und zu der erhaltenen Lösung die Substanz S sowie gegebenenfalls die weiteren Inhaltsstoffe des Wirkstoffkonzentrats, gegebenenfalls in gelöster Form zusetzt. Unter einem wässrigen Medium versteht man hier und im Folgenden Wasser, das einen Teil der gegebenenfalls vorhandenen sonstigen Bestandteile des Wirkstoffkonzentrats, z. B. Basen, Puffer, Konservierungsmittel etc., enthält. Das Lösen der Wirkstoffe der Formeln I und II kann gemeinsam oder sukzessive in einer Apparatur oder in getrennten Apparaturen erfolgen, wobei man in letzterem Fall die erhaltenen wässrigen Lösungen vereinigt. Häufig wird man zum Lösen der Wirkstoffe so vorgehen, dass man den Wirkstoff I in Wasser suspendiert und durch Zugabe einer Base oder eines Puffers den pH-Wert auf pH > 7, insbesondere pH ≥ 8, z. B. auf pH 8 bis pH 10, insbesondere auf pH 8 bis pH 9 einstellt, wobei der Wirkstoff der Formel I in Lösung geht. Die so erhaltene Lösung vermischt man dann mit einer wässrigen Lösung der Benzoesäureverbindung II bzw. mit einer wässrigen Lösung eines Salzes der Benzoesäureverbindung II, z. B. eines Alkalimetall-Salzes oder eines Ammoniumsalzes. Alternativ kann man auch so vorgehen, dass man ein Gemisch aus Wirkstoff I und Benzoesäureverbindung II bzw. eines der vorgenannten Salze der Benzoesäureverbindung II in Wasser suspendiert und dann durch Zugabe einer Base oder eines Puffers den pH-Wert der Suspension auf den oben angegebenen Bereich einstellt, wobei die Wirkstoffe der Formeln I und II in Lösung gehen. Anschließend gibt man zu den so erhaltenen Lösungen die übrigen Bestandteile des Wirkstoffkonzentrats und homogenisiert in üblicher Weise, z. B. durch Rühren, Ultraschall.

Die so erhaltenen wässrigen Wirkstoffkonzentrate eignen sich in besonderer Weise zur Bekämpfung einer Vielzahl unerwünschter Pflanzen. Die erfindungsgemäßen Wirkstoffkonzentrate eignen sich zur Bekämpfung von unerwünschtem Pflanzenwuchs auf Nichtkulturflächen sehr gut, besonders bei hohen Aufwandmengen. In Getreidekulturen wie Weizen, Roggen, Gerste, Hirse, Hafer oder Triticale sowie in Mais wirken sie gegen Unkräuter und Schadgräser, ohne die Kulturpflanzen nennenswert zu schädigen. Dieser Effekt tritt vor allem bei niedrigen Aufwandmengen auf. Die erfindungsgemäßen Wirkstoffkonzentrate eignen sich insbesondere für die Beseitigung von Schadpflanzen in Mais. In Abhängigkeit von der jeweiligen Applikationsmethode können die erfindungsgemäßen Wirkstoffkonzentrate noch in anderen Kulturpflanzen zur Beseitigung unerwünschter Pflanzen eingesetzt werden.

Darüber hinaus können die Wirkstoffkonzentrate auch in Kulturen, die durch Züchtung einschließlich gentechnischer Methoden gegen die Wirkung von Herbiziden tolerant sind, verwandt werden.

Die Applikation der Wirkstoffkonzentrate erfolgt in der Regel in Form einer wässrigen Spritzbrühe. Hierzu werden die erfindungsgemäßen Wirkstoffkonzentrate, abhängig von der Aufwandmenge, auf ein Vielfaches ihres Volumens, beispielsweise auf das 10-bis 10000-fache, insbesondere auf das 20- bis 1000 fache mit Wasser verdünnt. Die Wirkstoffkonzentration (Gesamtmenge an Wirkstoff) in der Spritzbrühe liegt dann typischerweise im Bereich von 5 mg/l bis 5 g/l, insbesondere 0,01 bis 1 g/l.

Die Applikation kann im Vorauflauf-, im Nachauflaufverfahren oder zusammen mit dem Saatgut einer Kulturpflanze erfolgen. Es besteht auch die Möglichkeit, die in den Wirkstoffkonzentraten enthaltenen Wirkstoffe der Formeln I und II dadurch zu applizieren, dass man unter Verwendung der erfindungsgemäßen Wirkstoffkonzentrate Saatgut einer Kulturpflanze mit den Wirkstoffe der Formeln I und II behandelt und das so behandelte Saatgut ausbringt. Sind die Wirkstoffe für gewisse Kulturpflanzen weniger verträglich, so können Ausbringungstechniken angewandt werden, bei welchen die unter Verwendung der Wirkstoffkonzentrate hergestellten Applikationsformen mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen, bezogen auf die Gesamtmenge an Wirkstoff I und II betragen je nach Bekämpfungsziel, Jahreszeit, Zielpflanzen und Wachstumsstadium 0.001 bis 3.0, vorzugsweise 0,005 bis 0,5 kg/ha.

Zur Verbreiterung des Wirkungsspektrums und zur Erzielung synergistischer Effekte können die Wirkstoffkonzentrate vor dem Ausbringen mit zahlreichen Vertretern anderer herbizider oder wachstumsregulierender Wirkstoffgruppen gemischt und gemeinsam ausgebracht werden, beispielsweise im Tank-Mix. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexan-dione, 2-Hetaroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximether-Derivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Wirkstoffkonzentrate vor dem Ausbringen auch noch mit weiteren Pflanzenschutzmitteln zu vermischen und gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### A Herstellungsbeispiele

### Beispiele 1 bis 11: Herstellung eines erfindungsgemäßen Wirkstoffkonzentrats (allgemeine Vorschrift)

50 g Topramezon (Wirkstoff der allgemeinen Formel I, worin in R¹ und R⁵ für Methyl, R² für 4,5-Dihydrooxazol-3-yl, R³ für Methylsulfonyl, R⁴ und R⁶ für Wasserstoff stehen) und 160 g Dicamba (Wirkstoff der Formel II, worin R⁷ für Methoxy und R⁸ für Wasserstoff stehen), wurden in 300 ml Wasser suspendiert. Man stellte den pH-Wert der Suspension durch Zugabe von 40 Gew.-%iger, wässriger Kalilauge auf pH-8,5 ein, gab zu der erhaltenen Lösung 300 g der jeweiligen Substanz S, gegebenenfalls in Form einer wässrigen Mischung, und Wasser ad 1 L und homogenisierte unter Rühren für 2 h. Man erhielt auf diese Weise eine klare Lösung, die 50 g/l Topramezon, 160 g/l Dicamba und 300 g der jeweiligen Substanz S enthielt.

Es wurden die folgenden Substanzen S eingesetzt:
S1: EO/PO-Triblock-Copolymerisat mit OH-Endgruppen, einem Molekulargewicht von 3100 Dalton (Zahlenmittel) und einem EO-Anteil von 42 Gew.-%.
S2: 2-Ethylhexylpolyglucosid mit 1,6 Glucoseeinheiten
S3: Polyethoxylat der Formel CH₃-O-(C₂H₄-O)₁₁-NH₂
S4: Ethoxyliertes Polyimin mit einem Ethoxilierungsgrad von 7 EO-Gruppen pro Stickstoffatom einem Molekulargewicht von etwa 14000 (Zahlenmittel) und einem Gewichtsanteil an EO-Gruppen von etwa 82 Gew.-%.
S5: Ethoxylat-co-Propoxylat der Formel R-O-(EO)ₓ(PO)_{y}H, worin EO und PO die zuvor genannten Bedeutungen aufweisen, R für lineares C₁₃-C₁₅-Alkyl steht steht, y für 23 steht und x 10 bedeutet.
S6: Ethoxylat der Formel R-O-(EO)ₓH, worin EO die zuvor genannten Bedeutungen aufweist, R für verzweigtes C₁₀-Alkyl steht und x 7 bedeutet (Lutensol ON 70).
S7: Ethoxylat-co-Propoxylat der Formel R-O-[(PO)_{y}(EO)ₓ]H, worin EO und PO statistisch angeordnet sind und die zuvor genannten Bedeutungen aufweisen, R für lineares C₉-C₁₁-Alkyl steht, y für 2 steht und x 7,5 bedeutet.
S8: Ethoxylat-co-Propoxylat der Formel R-O-(EO)ₓ(PO)_{y}H, worin EO und PO die zuvor genannten Bedeutungen aufweisen, R für verzweigtes C₁₃-Alkyl steht, y für 3 steht und x 6 bedeutet.
S9: Ethoxylat der Formel R-O-(EO)ₓH, worin EO die zuvor genannten Bedeutungen aufweist, R für verzweigtes C₁₃-Alkyl steht und x 5 bedeutet (Lutensol TO 5).
S10: Ethoxylat der Formel R-O-(EO)ₓH, worin EO die zuvor genannten Bedeutungen aufweist, R für verzweigtes C₁₀-Alkyl steht und x 3 bedeutet (Lutensol ON 30).
S11: Ethoxylat der Formel R-O-(EO)ₓH, worin EO die zuvor genannten Bedeutungen aufweist, R für verzweigtes C₁₀-Alkyl steht und x 5 bedeutet (Lutensol ON 50).

### B Untersuchung der Anwendungseigenschaften

Die erfindungsgemäßen Wirkstoffkonzentrate zeigten nach 2-wöchiger Lagerung bei 54 °C keine sichtbaren Veränderungen.

Die Bestimmung der Schaumbildungsneigung erfolgte nach Ross-Miles (ASTM-D 1173 53). Sie war insbesondere bei der mit S 5 formulierten Zubereitung gering.

### C Untersuchung der herbiziden Wirkung

### 1. Herbizide Wirkung gegenüber Schadgräsern

Die herbizide Wirkung der erfindungsgemäßen Wirkstoffkonzentrate gegen grasartige Schadpflanzen ließ sich durch die folgenden Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die mit Wasser auf die gewünschte Anwendungskonzentration verdünnten Wirkstoffkonzentrate direkt nach Einsaat mittels fein verteilender Düsen in der angegebenen Aufwandmenge aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den mit Wasser auf die gewünschte Anwendungskonzentration (ca. 66 bis 525 mg Wirkstoff/L) verdünnten Wirkstoffkonzentraten behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Als Schadpflanzen (Unkräuter) wurden Gräser der folgenden Spezies untersucht: Digitaria sanguinalis (DIGSA), Echinochloa crus-galli (ECHCG), Panicum sp. (PANDI), Panicum milliaceum (PANMI), Setaria faberi (SETFA), Setaria italica (SETIT), Setaria lutescens (SETLU), Setaria viridis (SETVI).

In den Tabelle 1 sind die bei Nachauflaufbehandlung erzielten Resultate dargestellt (Schädigung 20 bzw. 21 Tage nach Behandlung).

**Tabelle 1**

| Substanz S | Dosis | Schadpflanze | | | | | | | | Ø |
|---|---|---|---|---|---|---|---|---|---|---|
| | | DIGSA | ECHCG | PANDI | SETVA | PANMI | SETIT | SETLU | SETVI | |
| S1 | A | 100 | 85 | 70 | 100 | 100 | 100 | 100 | 100 | 94 |
| | B | 90 | 30 | 30 | 100 | 100 | 98 | 85 | 100 | 79 |
| | C | 75 | 0 | 20 | 98 | 95 | 95 | 85 | 80 | 69 |
| | D | 20 | 0 | 0 | 75 | 80 | 30 | 40 | 30 | 29 |
| S2 | A | 95 | 50 | 65 | 100 | 100 | 100 | 100 | 100 | 89 |
| | B | 50 | 20 | 30 | 100 | 98 | 98 | 95 | 100 | 74 |
| | C | 0 | 0 | 0 | 90 | 90 | 85 | 65 | 85 | 52 |
| | D | 0 | 0 | 0 | 50 | 85 | 30 | 40 | 30 | 29 |
| S3 | A | 95 | 30 | 75 | 100 | 98 | 100 | 95 | 98 | 86 |
| | B | 65 | 0 | 30 | 98 | 95 | 98 | 50 | 95 | 66 |
| | C | 10 | 0 | 0 | 85 | 90 | 90 | 30 | 80 | 48 |
| | D | 0 | 0 | 0 | 65 | 80 | 30 | 0 | 30 | 26 |
| S4 | A | 90 | 50 | 70 | 100 | 95 | 100 | 95 | 100 | 88 |
| | B | 60 | 20 | 40 | 98 | 95 | 98 | 90 | 100 | 75 |
| | C | 30 | 0 | 0 | 98 | 90 | 40 | 65 | 85 | 51 |
| | D | 0 | 0 | 0 | 50 | 75 | 20 | 30 | 40 | 27 |
| S5 | A | 100 | 95 | 75 | 100 | 100 | 100 | 100 | 100 | 96 |
| | B | 95 | 70 | 40 | 98 | 100 | 100 | 90 | 98 | 86 |
| | C | 50 | 30 | 20 | 98 | 90 | 90 | 85 | 40 | 63 |
| | D | 20 | 0 | 0 | 60 | 80 | 30 | 50 | 20 | 33 |
| S6 | A | 100 | 90 | 70 | 100 | 100 | 100 | 100 | 100 | 95 |
| | B | 90 | 75 | 30 | 98 | 98 | 100 | 95 | 100 | 86 |
| | C | 40 | 30 | 0 | 98 | 95 | 75 | 70 | 90 | 62 |
| | D | 0 | 0 | 0 | 90 | 80 | 30 | 40 | 40 | 35 |
| S6 | A | 98 | 98 | 25 | 100 | 98 | 98 | 98 | 98 | 86 |
| | B | 85 | 25 | 20 | 98 | 95 | 95 | 85 | 95 | 67 |
| | C | 75 | 10 | 0 | 98 | 70 | 50 | 10 | 70 | 48 |
| | D | 40 | 0 | 0 | 35 | 40 | 40 | 10 | 30 | 23 |
| S7 | A | 95 | 98 | 70 | 100 | 98 | 100 | 100 | 98 | 89 |
| | B | 90 | 40 | 10 | 98 | 95 | 85 | 95 | 95 | 67 |
| | C | 45 | 0 | 0 | 90 | 70 | 60 | 30 | 60 | 41 |
| | D | 40 | 0 | 0 | 35 | 40 | 40 | 10 | 30 | 23 |
| S8 | A | 98 | 85 | 15 | 100 | 98 | 98 | 98 | 98 | 80 |
| | B | 85 | 12 | 10 | 90 | 80 | 90 | 80 | 98 | 64 |
| | C | 65 | 0 | 5 | 0 | 20 | 70 | 10 | 15 | 22 |
| | D | 20 | 0 | 0 | 0 | 20 | 25 | 10 | 15 | 11 |
| S9 | A | 98 | 30 | 65 | 98 | 90 | 45 | 85 | 95 | 76 |
| | B | 90 | 10 | 20 | 100 | 75 | 70 | 70 | 30 | 37 |
| | C | 50 | 0 | 0 | 75 | 65 | 55 | 15 | 40 | 34 |
| | D | 45 | 0 | 0 | 40 | 40 | 30 | 10 | 25 | 22 |
| S10 | A | 98 | 80 | 75 | 98 | 98 | 90 | 98 | 98 | 84 |
| | B | 80 | 20 | 10 | 98 | 85 | 85 | 80 | 98 | 63 |
| S11 | A | 98 | 95 | 70 | 98 | 98 | 90 | 98 | 98 | 89 |
| | B | 90 | 20 | 10 | 95 | 85 | 90 | 65 | 90 | 70 |
| | C | 60 | 0 | 0 | 80 | 60 | 70 | 40 | 35 | 39 |
| | D | 20 | 0 | 0 | 30 | 40 | 40 | 20 | 25 | 19 |
| keine | A' | 80 | 15 | 10 | 90 | 80 | 85 | 80 | 90 | 60 |
| | B' | 20 | 0 | 5 | 70 | 70 | 70 | 50 | 70 | 40 |
| | C' | 15 | 0 | 5 | 70 | 60 | 60 | 20 | 65 | 34 |
| | D' | 15 | 0 | 0 | 30 | 40 | 40 | 10 | 20 | 18 |

| | | | | | | | | | | |
|---|---|---|---|---|---|---|---|---|---|---|
| A: 25 g/ha Topramezon, 80 g/ha Dicamba, 150 g/ha Substanz S B: 12,5 g/ha Topramezon, 40 g/ha Dicamba, 75 g/ha Substanz S C: 6,25 g/ha Topramezon, 20 g/ha Dicamba, 37,5 g/ha Substanz S D: 3,13 g/ha Topramezon, 10 g/ha Dicamba, 18,8 g/ha Substanz S A': 25 g/ha Topramezon, 80 g/ha Dicamba, B': 12,5 g/ha Topramezon, 40 g/ha Dicamba, C': 6,25 g/ha Topramezon, 20 g/ha Dicamba, D': 3,13 g/ha Topramezon, 10 g/ha Dicamba, | | | | | | | | | | |

Die herbizide Wirkung erfindungsgemäß formulierter Konzentrate gegenüber grasartigen Schadpflanzen liegt bei gleicher Aufwandmenge deutlich über der Wirkung eines Konzentrats, das ohne Zusatz oberflächenaktiver Substanzen. Insbesondere bei Setaria-Arten war eine vollständige Kontrolle möglich.

### 2. Herbizide Wirkdung gegenüber nicht grasartigen Schadpflanzen

In zu 1. analoger Weise wurde die Wirksamkeit erfindungsgemäßer Formulierungen gegenüber den nicht grasartigen Schadpflanzen Avena fatua, Sorghum bicolor untersucht.

In den Tabelle 2 sind die bei Nachauflaufbehandlung erzielten Resultate dargestellt (untersucht 20 bzw. 21 Tage nach Behandlung).

**Tabelle 2**

| Substanz S (Klasse) | Dosis | Zielpflanze | |
|---|---|---|---|
| | | Avena fatua | Sorghum bicolor |
| S1 | A | 50 | 50 |
| | B | 40 | 40 |
| | C | 30 | 30 |
| | D | 20 | 20 |
| S2 | A | 40 | 20 |
| | B | 30 | 0 |
| | C | 20 | 0 |
| | D | 10 | 0 |
| S3 | A | 50 | 30 |
| | B | 30 | 20 |
| | C | 20 | 0 |
| | D | 10 | 0 |
| S4 | A | 50 | 25 |
| | B | 30 | 0 |
| | C | 20 | 0 |
| | D | 10 | 0 |
| S5 | A | 40 | 75 |
| | B | 30 | 30 |
| | C | 20 | 0 |
| | D | 10 | 0 |
| S6 | A | 40 | 25 |
| | B | 30 | 20 |
| | C | 10 | 0 |
| | D | 0 | 0 |
| S7 | A | 40 | 70 |
| | B | 5 | 10 |
| | C | 15 | 0 |
| | D | 5 | 0 |
| S8 | A | 30 | 15 |
| | B | 25 | 10 |
| | C | 10 | 5 |
| | D | 5 | 0 |
| S9 | A | 30 | 65 |
| | B | 15 | 20 |
| | C | 10 | 0 |
| | D | 5 | 0 |
| S10 | A | 20 | 75 |
| | B | 10 | 10 |
| | C | 0 | 5 |
| | D | 0 | 0 |
| S11 | A | 60 | 70 |
| | B | 85 | 10 |
| | C | 5 | 0 |
| | D | 0 | 0 |
| kein | A' | 70 | 10 |
| | B' | 10 | 5 |
| | C' | 10 | 5 |
| | D' | 5 | 0 |

| | | | |
|---|---|---|---|
| A: 25 g/ha Topramezon, 80 g/ha Dicamba, 150 g/ha Substanz S, bzw. A': 25 g/ha Topramezon, 80 g/ha Dicamba, B: 12,5 g/ha Topramezon, 40 g/ha Dicamba, 75 g/ha Substanz S, bzw. B: 12,5 g/ha Topramezon, 40 g/ha Dicamba C: 6,25 g/ha Topramezon, 20 g/ha Dicamba, 37,5 g/ha Substanz S, bzw. C: 6,25 g/ha Topramezon, 20 g/ha Dicamba D: 3,13 g/ha Topramezon, 10 g/ha Dicamba, 18,8 g/ha Substanz S, bzw. D: 3,13 g/ha Topramezon, 10 g/ha Dicamba | | | |

### 3. Herbizide Wirkung gegenüber Nutzpflanzen

In zu 1. analoger Weise wurde die Wirksamkeit erfindungsgemäßer Wirkstoffkonzentrate gegenüber den Nutzpflanzen Zea mays (ZEAMX) der Sorten "Dea" und "Helix" untersucht.

Die Behandlung der Pflanzen erfolgte im Nachauflauf. Die Schädigung der Pflanzen wurde am Tag 6 bzw. 8 (Messung A) und am Tag 20 bzw. 21 (Messung B) nach der Behandlung ermittelt. Ansonsten wurde wie unter Beispiel 1 beschrieben vorgegangen.

In den Tabelle 3 sind die bei Nachauflaufbehandlung erzielten Resultate dargestellt.

**Tabelle 3**

| Substanz S | Dosis [MI/ha] | Zielpflanze und Tag | | | |
|---|---|---|---|---|---|
| | | ZEAMX "Dea" | | ZEAMX "Helix" | |
| | | A | B | A | B |
| S1 | A | 5 | 15 | 5 | 0 |
| | B | 5 | 0 | 5 | 0 |
| | C | 0 | 0 | 5 | 0 |
| | D | 0 | 0 | 0 | 0 |
| S2 | A | 5 | 0 | 5 | 0 |
| | B | 0 | 0 | 5 | 0 |
| | C | 0 | 0 | 0 | 0 |
| | D | 0 | 0 | 0 | 0 |
| S3 | A | 5 | 0 | 5 | 0 |
| | B | 5 | 0 | 5 | 0 |
| | C | 0 | 0 | 0 | 0 |
| | D | 0 | 0 | 0 | 0 |
| S4 | A | 5 | 0 | 5 | 0 |
| | B | 0 | 0 | 5 | 0 |
| | C | 0 | 0 | 0 | 0 |
| | D | 0 | 0 | 0 | 0 |
| S5 | A | 5 | 5 | 0 | 5 |
| | B | 5 | 0 | 0 | 0 |
| | C | 5 | 0 | 0 | 0 |
| | D | 0 | 0 | 0 | 0 |
| S6 | A | 0 | 0 | 0 | 5 |
| | B | 0 | 0 | 0 | 0 |
| | C | 0 | 0 | 0 | 0 |
| | D | 0 | 0 | 0 | 0 |
| S7 | A | 0 | 0 | 0 | 0 |
| | B | 0 | 0 | 0 | 0 |
| | C | 0 | 0 | 0 | 0 |
| | D | 0 | 0 | 0 | 0 |
| S8 | A | 5 | 0 | 5 | 0 |
| | B | 0 | 0 | 0 | 0 |
| | C | 0 | 0 | 0 | 0 |
| | D | 0 | 0 | 0 | 0 |
| S9 | A | 0 | 5 | 0 | 0 |
| | B | 0 | 0 | 0 | 0 |
| | C | 0 | 0 | 0 | 0 |
| | D | 0 | 0 | 0 | 0 |
| S10 | A | 0 | 0 | 5 | 0 |
| | B | 0 | 0 | 0 | 0 |
| | C | 0 | 0 | 0 | 0 |
| | D | 0 | 0 | 0 | 0 |
| S11 | A | 0 | 0 | 5 | 0 |
| | B | 0 | 0 | 0 | 0 |
| | C | 0 | 0 | 0 | 0 |
| | D | 0 | 0 | 0 | 0 |
| Keine Substanz S | A' | 0 | 0 | 0 | 0 |
| | B' | 0 | 0 | 0 | 0 |
| | C' | 0 | 0 | 0 | 0 |
| | D' | 0 | 0 | 0 | 0 |

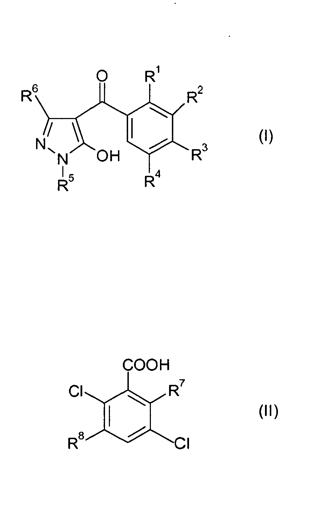

## Patentansprüche

1. Wässriges Wirkstoffkonzentrat, enthaltend in gelöster Form:
a) mindestens eine 4-Benzoyl-substituierte. Pyrazolverbindung der Formel I 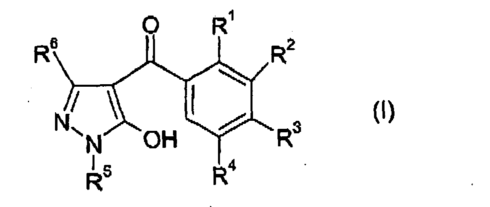 worin
R¹, R³ unabhängig voneinander für Wasserstoff, Halogen, Methyl, Halogenmethyl, Methoxy, Halogenmethoxy, Methylfihio, Methylsulfinyl oder Methylsulfonyl stehen;
R2 für einen 5-gliedrigen heterocyclischen Rest steht, der unsubstituiert ist oder 1, 2, 3 oder 4 Substituenten trägt, die unter Halogen, C₁-C₆-Alkyl, C₁-C₄-Akoxy, C₁-C₄-Halogenalkyl, C₁-C₄-Halogenalkoxy und C₁-C₄-Alkylthio ausgewählt sind;
R⁴ Wasserstoff, Halogen oder Methyl bedeutet;
R⁵ _{C}1-C₆-Alkyl, C₃-C₈-Cycloalkyl oder C₃-C₆-Cycloalkylmethyl bedeutet; und
R⁶ für Wasserstoff oder C₁-C₄-Alkyl steht;
oder eines seiner landwirtschaftlich verwendbaren Salze;
b) mindestens eine Benzoesäureverbindung der Formel II 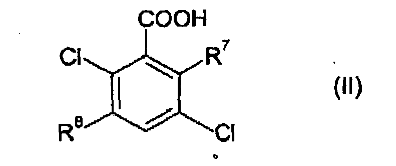 worin
R⁷ Wasserstoff, Halogen, Hydroxy oder Methoxy und
R⁸ Wasserstoff, Halogen oder Amino bedeuten;
oder eines seiner landwirtschaftlich verwendbaren Salze; und
c) mindestens eine nichtionische oberflächenaktive Substanz S, ausgewählt unter Polyetherverbindungen, die von Ethylenoxid abgeleitete Wiederholungseinheiten aufweisen, Alkylpolyglykosiden und Gemischen davon
worin das Gewichtsverhältnis der Gesamtwirkstoffmenge von Pyrazolverbindung der Formel I und Benzoesäureverbindung der Formel II zu oberflächenaktiver Substanz S im Bereich von 1 : 10 bis 3 : 1 liegt,
wobei die Polyetherverbindung wenigstens eine Polyethergruppe der Formel III
R^{x}-[(EO)ₓ(AO)_{y}]- (III)
aufweist, worin
EO für -CH₂-CH₂-O- steht;
AO für -CHR^{a}-CR^{b}R^{c}-O- steht;
R^{x} für Wasserstoff, C₁-C₁₀-Alkyl, C₅-C₁₀-Cycloalkyl, Benzoyl oder C₁-C₂₀-Alkylcarbonyl steht und über das Sauerstoffatom einer EO-Gruppe oder einer AO-Gruppe gebunden ist;
x eine ganze Zahl bedeutet, deren Zahlenmittel im Bereich von 1 bis 150 liegt;
y eine ganze Zahl bedeutet, deren Zahlenmittel im Bereich von 0 bis 150 liegt, wobei das Zahlenmittel der Summe von x und y im Bereich von 5 bis 150 liegt;
R^{a}, R^{b} unabhängig voneinander für Wasserstoff oder Methyl stehen; und
R^{c} Wasserstoff, C₁-C₄-Alkyl oder Phenyl bedeutet
wobei wenigstens einer der Reste R^{a}, R^{b} und R^{c} von Wasserstoff verschieden ist, und wobei die Polyetherverbindung ausgewählt ist unter
- Ethylenoxyd-Propylenoxyd-Copolymeren,
- Polyetherverbindungen, in denen die wenigstens eine Polyethergruppe der Formel III über ein Sauerstoff-, Schwefel- oder Stickstoffatom kovalent an einen Kohlenwasserstoffrest mit 8 bis 40 Kohlenstoffatomen, der gegebenenfalls noch 1 oder 2 Carbonyloxygruppen und/oder 1, 2, 3 oder 4 OH-Gruppen aufweist, gebunden ist,
und Gemischen davon.

2. Wirkstoffkonzentrat nach Anspruch 1,worin die nichtionische oberflächenaktive Substanz S einen HL.B-Wert nach Griffin im Bereich von 1,5 bis 19,5 aufweist.

3. Wirkstoffkonzentrat nach Anspruch 1, worin die nichtionische oberflächenaktive Substanz S einen modifizierten HLB-Wert im Bereich von 5 bis 19,5 aufweist.

4. Wirkstoffkonzentrat nach Anspruch 1, wobei die Polyetherverbindung ausgewählt ist unter Polyethoxylaten und Poly(ethoxylat-co-propoxylat)en von C₈-C₂₂-Alkanolen, Polyethoxylaten und Poly(ethoxylat-co-propoxylat)en von Fettsäuren, Polyethoxylaten und Poly(ethoxylat-co-propoxylat)en von Fettaminen, Polyethoxylaten und Poly(ethoxylat-co-propoxylat)en von Mono- und Diglyceriden aliphatischer C₈-C₂₂-Monocarbonsäuren, Polyethoxylaten und Poly(ethoxylat-co-propoxylat)en von Sorbitanestern aliphatischer C₈-C₂₂-Monocarbonsäuren, Polyethoxylaten und Poly(ethoxylat-co-propoxylat)en von Alkylphenolen, Polyethoxylaten und Poly(ethoxylat-co-propoxylat)en von Di- und Tristyrylphenolen, Polyethoxylaten und Poly(ethoxylat-co-propoxylat)en von Alkylpolyglykosiden und Gemischen davon.

5. Wirkstoffkonzentrat nach einem der Ansprüche 1 bis 4, wobei die Polyetherverbindung einen Polyetherrest der Formel III aufweist, der über ein Sauerstoff an einen C₈-C₂₂-Alkylrest gebunden ist, wobei R^{x} in Formel III für Wasserstoff steht, EO für CH₂CH₂O steht, AO für CH₂CH(CH₃)O steht, x eine Zahl bedeutet, deren Zahlenmittel im Bereich von 3 bis 49 liegt, y eine Zahl bedeutet, deren Zahlenmittel im Bereich von 1 bis 47 liegt, und das Zahlenmittel der Summe x+y im Bereich von 5 bis 50 liegt.

6. Wirkstoffkonzentrat nach Anspruch 5, worin die Polyetherverbindung ausgewählt ist unter Verbindungen der allgemeinen Formel IV
R¹¹-O-[(EO)ₓ(AO)_{y}]-R^{x},
worin R¹¹ für Alkyl mit 8 bis 30 C-Atomen steht und worin x, y, EO. AO und R^{x} die in Anspruch 4 angegebenen Bedeutungen aufweisen.

7. Wirkstoffkonzentrat nach einem der vorhergehenden Ansprüche, enthaltend die Komponenten a) und b) in Form ihrer gelösten Alkalimetall- oder Ammoniumsalze.

8. Wirkstoffkonzentrat nach einem der vorigen Ansprüche, enthaltend eine 4-Benzoyl-substituierte Pyrazolverbindung der Formel I, worin R² ausgewählt unter Thiazol-2-yl, Thiazol-4-yl, Thiazol-5-yl, Isoxazol-3-yl, Isoxazol-4-yl, Isoxazol-5-yl, 4,5-Dihydroisoxazol-3-yl, 4,5-Dihydroisoxazol-4-yl und 4,5-Dihydrisoxazol-5-yl, wobei die vorgenannten Reste unsubstituiert sind oder 1 oder 2 Methylgruppen als Substituenten tragen können.

9. Wirkstoffkonzentrat nach Anspruch 8, wobei in Formel I R¹ und R⁵ jeweils Methyl bedeuten, R² für 4,5-Dihydroisoxazol-3-yl steht, R⁴ Methylsulfonyl bedeutet und R⁶ Wasserstoff bedeutet.

10. Wirkstoffkonzentrat nach einem der vorigen Ansprüche, enthaltend eine Benzoesäureverbindung der Formel II, worin R⁷ Methoxy und R⁸ Wasserstoff bedeuten.

11. Wirkstoffkonzentrat nach einem der vorigen Ansprüche, enthaltend
- 10 bis 100 g/l einer 4-Benzoyl-substituierten Pyrazolverbindung der Formel I,
- 50 bis 250 g/l einer Benzoesäureverbindung der Formel II,
- 100 bis 500 g/l wenigstens einer nicht-ionischen, oberflächenaktiven Substanz S und
- Wasser.

12. Wirkstoffkonzentrat nach einem der vorigen Ansprüche, enthaltend die 4-Benzoyl-substituierte Pyrazolverbindung der Formel I und die Benzoesäureverbindung der Formel II in einem Gewichtsverhältnis von 1 : 25 bis 2 : 1.

13. Verwendung eines Wirkstoffkonzentrats nach einem der Ansprüche 1 bis 12 zur Bekämpfung unerwünschten Pflanzenwuchses.

14. Verfahren zur Bekämpfung unerwünschten Pflanzenwuchses, **dadurch gekennzeichnet, dass** man eine wässrige Spritzbrühe durch Verdünnen eines Wirkstoffkonzentrats nach einem der Ansprüche 1 bis 12 herstellt und diese auf Pflanzen, deren Samen und/oder deren Lebensraum einwirken lässt.

15. Verfahren nach Anspruch 14, **dadurch gekennzeichnet, dass** man die Blätter der unerwünschten Pflanzen mit der wässrigen Spritzbrühe behandelt.

16. Verwendung einer nichtionischen oberflächenaktiven Substanz S, wie in einem der Ansprüche 1 bis 6 definiert, als Adjuvans zur Herstellung eines wässrigen Wirkstoffkonzentrats nach einem der Ansprüche 1 bis 12.

## Claims

1. An aqueous active compound concentrate comprising, in dissolved form:
a) at least one 4-benzoyl-substituted pyrazole compound of the formula I 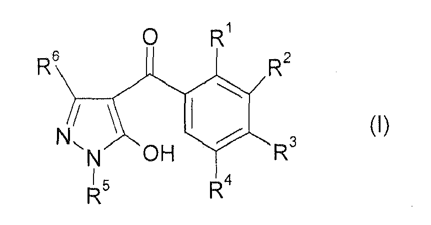 in which
R¹, R³ independently of one another are hydrogen, halogen, methyl, halomethyl, methoxy, halomethoxy, methylthio, methylsulfinyl or methylsulfonyl;
R² is a 5-membered heterocyclic radical which is unsubstituted or carries 1, 2, 3 or 4 substituents selected from the group consisting of halogen, C₁-C₆-alkyl, C₁-C₄-alkoxy, C₁-C₄-haloalkyl, C₁-C₄-haloalkoxy and C₁-C₄-alkylthio;
R⁴ is hydrogen, halogen or methyl;
R⁵ is C₁-C₆-alkyl, C₃-C₆-cycloalkyl or C₃-C₆-cycloalkylmethyl; and
R⁶ is hydrogen or C₁-C₄-alkyl;
or one of its agriculturally useful salts;
b) at least one benzoic acid compound of the formula II 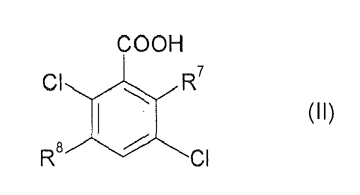 in which
R⁷ is hydrogen, halogen, hydroxyl or methoxy and
R⁸ is hydrogen, halogen or amino;
or one of its agriculturally useful salts; and
c) at least one nonionic surfactant S, selected from polyether compounds having repeat units derived from ethylene oxide, alkylpolyglycosides and mixtures thereof
in which the weight ratio of the total amount of active compound of pyrazole compound of the formula I and benzoic acid compound of the formula II to surfactant S is in the range of from 1:10 to 3:1,
the polyether compound having at least one polyether group of the formula III
R^{x}-[(EO)ₓ (AO)y]- (III)
in which
EO is -CH₂-CH₂-O-;
AO is -CHR^{a}-CR^{b}R^{c}-O-;
R^{x} is hydrogen, C₁-C₁₀-alkyl, C₅-C₁₀-cycloalkyl, benzoyl or C₁-C₂₀-alkylcarbonyl and is attached via the oxygen atom of an EO group or an AO group;
x is an integer whose number average is in the range of from 1 to 150;
y is an integer whose number average is in the range of from 0 to 150, the number average of the sum of x and y being in the range of from 5 to 150;
R^{a}, R^{b} independently of one another are hydrogen or methyl; and
R^{c} is hydrogen, C₁-C₄-alkyl or phenyl
where at least one of the radicals R^{a}, R^{b} and R^{c} is different from hydrogen,
and where the polyether compound is selected from
- ethylene oxide/propylene oxide copolymers,
- polyether compounds in which the at least one polyether group of the formula III is attached covalently via an oxygen, sulfur or nitrogen atom to a hydrocarbon radical having 8 to 40 carbon atoms, and which optionally also has 1 or 2 carbonyloxy groups and/or 1, 2, 3 or 4 OH groups,
and mixtures thereof.

2. The active compound concentrate according to claim 1 in which the nonionic surfactant S has an HLB according to Griffin in the range of from 1.5 to 19.5.

3. The active compound concentrate according to claim 1 in which the nonionic surfactant S has a modified HLB in the range of from 5 to 19.5.

4. The active compound concentrate according to claim 1 where the polyether compound is selected from polyethoxylates and poly(ethoxylate-co-propoxylate)s of C₈-C₂₂-alkanols, polyethoxylates and poly(ethoxylate-co-propoxylate)s of fatty acids, polyethoxylates and poly(ethoxylate-co-propoxylate)s of fatty amines, polyethoxylates and poly(ethoxylate-co-propoxylate)s of mono- and diglycerides of aliphatic C₈-C₂₂-monocarboxylic acids, polyethoxylates and poly(ethoxylate-co-propoxylate)s of sorbitan esters of aliphatic C₈-C₂₂-monocarboxylic acids, polyethoxylates and poly(ethoxylate-co-propoxylate)s of alkylphenols, polyethoxylates and poly(ethoxylate-co-propoxylate)s of di- and tristyrylphenols, polyethoxylates and poly(ethoxylate-co-propoxylate)s of alkylpolyglycosides and mixtures thereof.

5. The active compound concentrate according to any of claims 1 to 4 where the polyether compound has a polyether radical of the formula III which is attached via oxygen to a C₈-C₂₂-alkyl radical, where R^{x} in formula III is hydrogen, EO is CH₂CH₂O, AO is CH₂CH(CH₃)O, x is a number whose number average is in the range of from 3 to 49, y is a number whose number average is in the range of from 1 to 47 and the number average of the sum x + y is in the range of from 5 to 50.

6. The active compound concentrate according to claim 5 in which the polyether compound is selected from compounds of the general formula IV
R¹¹-O-[(EO)ₓ(AO)y]-R^{x},
in which R¹¹ is alkyl having 8 to 30 carbon atoms and in which x, y, EO, AO and R^{x} have the meanings given in claim 1.

7. The active compound concentrate according to any of the preceding claims, comprising components a) and b) in the form of their dissolved alkali metal or ammonium salts.

8. The active compound concentrate according to any of the preceding claims, comprising a 4-benzoyl-substituted pyrazole compound of the formula I in which R² is selected from the group consisting of thiazol-2-yl, thiazol-4-yl, thiazol-5-yl, isoxazol-3-yl, isoxazol-4-yl, isoxazol-5-yl, 4,5-dihydroisoxazol-3-yl, 4,5-dihydroisoxazol-4-yl and 4,5-dihydroisoxazol-5-yl, where the radicals mentioned above are unsubstituted or may carry 1 or 2 methyl groups as substituents.

9. The active compound concentrate according to claim 8 where in formula I R¹ and R⁵ are each methyl, R² is 4,5-dihydroisoxazol-3-yl, R⁴ is methylsulfonyl and R⁶ is hydrogen.

10. The active compound concentrate according to any of the preceding claims, comprising a benzoic acid compound of the formula II in which R⁷ is methoxy and R⁸ is hydrogen.

11. The active compound concentrate according to any of the preceding claims, comprising
- from 10 to 100 g/l of a 4-benzoyl-substituted pyrazole compound of the formula I,
- from 50 to 250 g/l of a benzoic acid compound of the formula II,
- from 100 to 500 g/l of at least one nonionic surfactant S and
- water.

12. The active compound concentrate according to any of the preceding claims, comprising the 4-benzoyl-substituted pyrazole compound of the formula I and the benzoic acid compound of the formula II in a weight ratio of from 1:25 to 2:1.

13. The use of an active compound concentrate according to any of claims 1 to 12 for controlling unwanted vegetation.

14. A method for controlling unwanted vegetation, **characterized in that** an aqueous spray liquor is prepared by diluting an active compound concentrate according to any of claims 1 to 12 and allowing the spray liquor to act on plants, their seeds and/or their habitat.

15. The method according to claim 14, **characterized in that** the leaves of the unwanted plants are treated with the aqueous spray liquor.

16. The use of a nonionic surfactant S, as defined in any of claims 1 to 6, as adjuvant for preparing an aqueous active compound concentrate according to any of claims 1 to 12.

## Revendications

1. Concentré aqueux de substances actives, contenant sous forme dissoute :
a) au moins un composé de type pyrazole à substitution 4-benzoyle, de formule I 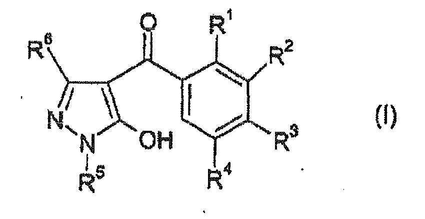 dans laquelle
R¹, R³ représentent, indépendamment l'un de l'autre, un atome d'hydrogène ou d'halogène, un groupe méthyle, halogénométhyle, méthoxy, halogénométhoxy, méthylthio, méthylsulfinyle ou méthylsulfonyle ;
R² représente un radical hétérocyclique à 5 chaînons, qui est non substitué ou porte 1, 2, 3 ou 4 substituants qui sont choisis parmi des atomes d'halogène et des groupes alkyle en C₁-C₆, alcoxy en C₁-C₄, halogénoalkyle en C₁-C₄, halogénoalcoxy en C₁-C₄ et alkyl(C₁-C₄)thio ;
R⁴ représente un atome d'hydrogène ou d'halogène ou le groupe méthyle ;
R⁵ représente un groupe alkyle en C₁-C₆, cycloalkyle en C₃-C₆ ou cycloalkyl(C₃-C₆)méthyle ; et
R⁶ représente un atome d'hydrogène ou un groupe alkyle en C₁-C₄ ;
ou un de ses sels utilisables en agriculture ;
b) au moins un composé de type acide benzoïque de formule II 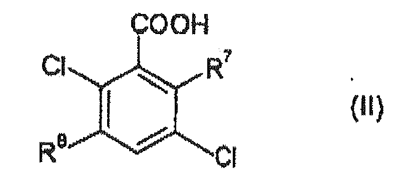 dans laquelle
R⁷ représente un atome d'hydrogène ou d'halogène, un groupe hydroxy ou méthoxy et
R⁸ représente un atome d'hydrogène ou d'halogène ou un groupe amino ;
ou un de ses sels utilisables en agriculture ; et
c) au moins une substance tensioactive non ionique S, choisie parmi des composés de type polyéther qui comportent des motifs répétitifs dérivés d'oxyde d'éthylène, des alkylpolyglycosides et des mélanges de ceux-ci
dans lequel le rapport pondéral de la quantité totale de substances actives du composé de type pyrazole de formule I et du composé de type acide benzoïque de formule II à la substance tensioactive S se situe dans la plage de 1:10 à 3:1,
le composé de type polyéther comportant au moins un groupe polyéther de formule III
R^{x}-[(EO)ₓ(AO)_{y}]- (III)
dans laquelle
EO représente -CH₂-CH₂-O- ;
AO représente -CHR^{a}-CR^{b}R^{c}-O-;
R^{x} représente un atome d'hydrogène, un groupe alkyle en C₁-C₁₀ , cycloalkyle en C₅-C₁₀, benzoyle ou alkyl (C₁-C₂₀) carbonyle et est lié par l'atome d'oxygène d'un groupe EO ou d'un groupe AO ;
x représente un nombre entier dont la moyenne en nombre se situe dans la plage de 1 à 150 ;
y représente un nombre entier dont la moyenne en nombre se situe dans la plage de 0 à 150, la moyenne en nombre de la somme de x et y se situant dans la plage de 5 à 150 ;
R^{a}, R^{b} représentent, indépendamment l'un de l'autre, un atome d'hydrogène ou le groupe méthyle ; et
R^{c} représente un atome d'hydrogène, un groupe alkyle en C₁-C₄ ou phényle,
au moins l'un des radicaux R^{a}, R^{b} et R^{c} étant différent d'un atome d'hydrogène et le composé de type polyéther étant choisi parmi
- des copolymères oxyde d'éthylène/oxyde de propylène,
- des composés de type polyéther dans lesquels au moins un groupe polyéther de formule III est lié par covalence via un atome d'oxygène, de soufre ou d'azote à un radical hydrocarboné ayant de 8 à 40 atomes de carbone, qui éventuellement comporte encore 1 ou 2 groupes carbonyloxy et/ou 1, 2, 3 ou 4 groupes OH, et des mélanges de ceux-ci.

2. Concentré de substances actives selon la revendication 1, dans lequel la substance tensioactive non ionique S présente une valeur HLB (rapport hydrophile-lipophile) selon Griffin dans la plage de 1,5 à 19,5.

3. Concentré de substances actives selon la revendication 1, dans lequel la substance tensioactive non ionique S présente une valeur HLB modifiée dans la plage de 5 à 19,5.

4. Concentré de substances actives selon la revendication 1, dans lequel le composé de type polyéther est choisi parmi des polyéthoxylates et copolymères éthoxylate/propoxylate d'alcanols en C₈-C₂₂, des polyéthoxylates et copolymères éthoxylate/ propoxylate d'acides gras, des polyéthoxylates et copolymères éthoxylate/propoxylate d'amines grasses, des polyéthoxylates et copolymères éthoxylate/propoxylate de mono- et diglycérides d'acides monocarboxyliques en C₈-C₂₂ aliphatiques, des polyéthoxylates et copolymères éthoxylate/propoxylate d'esters de sorbitanne d'acides monocarboxyliques en C₈-C₂₂ aliphatiques, des polyéthoxylates et copolymères éthoxylate/propoxylate d'alkylphénols, des polyéthoxylates et copolymères éthoxylate/propoxylate de di- et tristyrylphénols, des polyéthoxylates et copolymères éthoxylate/propoxylate d'alkylpolyglycosides et des mélanges de ceux-ci.

5. Concentré de substances actives selon l'une quelconque des revendications 1 à 4, dans lequel le composé de type polyéther comporte un radical polyéther de formule III, qui est lié par un atome d'oxygène à un radical alkyle en C₈-C₂₂, R^{x} dans la formule III représentant un atome d'hydrogène, EO représentant CH₂CH₂O, AO représentant CH₂CH(CH₃)O, x représentant un nombre dont la moyenne en nombre se situe dans la plage de 3 à 49, y représentant un nombre dont la moyenne en nombre se situe dans la plage de 1 à 47, et la moyenne en nombre de la somme x+y se situant dans la plage de 5 à 50.

6. Concentré de substances actives selon la revendication 5, dans lequel le composé de type polyéther est choisi parmi des composés de formule générale IV
R¹¹-O-[(EO)ₓ(AO)_{y}]-R^{x}
dans laquelle R¹¹ représente un nombre alkyle ayant de 8 à 30 atomes de carbone et dans laquelle x, y, EO, AO et R^{x} ont les significations données dans la revendication 1.

7. Concentré de substances actives selon l'une quelconque des revendications précédentes, contenant les composants a) et b) sous forme de leurs sels dissous de métaux alcalins ou d'ammonium.

8. Concentré de substances actives selon l'une quelconque des revendications précédentes, contenant un composé de type pyrazole à substitution 4-benzoyle de formule I, dans lequel R² est choisi parmi les groupes thiazol-2-yle, thiazol-4-yle, thiazol-5-yle, isoxazol-3-yle, isoxazol-4-yle, isoxazol-5-yle, 4,5-dihydroisoxazol-3-yle, 4,5-dihydro-isoxazol-4-yle et 4,5-dihydro-isoxazol-5-yle, les radicaux précités étant non substitués ou prouvant porter 1 ou 2 groupes méthyle en tant que substituants.

9. Concentré de substances actives selon la revendication 8, dans lequel R¹ et R⁵ dans la formule I représentent chacun un groupe méthyle, R² représente le groupe 4,5-dihydro-isoxazol-3-yle, R⁴ représente le groupe méthylsulfonyle et R⁶ représente un atome d'hydrogène.

10. Concentré de substances actives selon l'une quelconque des revendications précédentes, contenant un composé de type acide benzoïque de formule II, dans lequel R⁷ représente le groupe méthoxy et R⁸ représente un atome d'hydrogène.

11. Concentré de substances actives selon l'une quelconque des revendications précédentes, contenant
- 10 à 100 g/l d'un composé de type pyrazole à substitution 4-benzoyle de formule I,
- 50 à 250 g/l d'un composé de type acide benzoïque de formule II,
- 100 à 500 g/l d'au moins une substance tensioactive non ionique S et
- de l'eau.

12. Concentré de substances actives selon l'une quelconque des revendications précédentes, contenant le composé de type pyrazole à substitution 4-benzoyle de formule I et le composé de type acide benzoïque de formule II en un rapport pondéral de 1:25 à 2:1.

13. Utilisation d'un concentré de substances actives selon l'une quelconque des revendications 1 à 12, pour la lutte contre une croissance de plantes indésirables.

14. Procédé pour combattre une croissance de plantes indésirables, **caractérisé en ce qu'**on prépare une bouillie aqueuse à pulvériser, par dilution d'un concentré de substances actives selon l'une quelconque des revendications 1 à 12, et on la laisse agir sur des plantes, leurs graines et/ou leur habitat.

15. Procédé selon la revendication 14, **caractérisé en ce qu'**on traite les feuilles des plantes indésirables par la bouillie aqueuse à pulvériser.

16. Utilisation d'une substance tensioactive non ionique S, telle que définie dans l'une quelconque des revendications 1 à 6, en tant qu'adjuvant pour la préparation d'un concentré aqueux de substances actives selon l'une quelconque des revendications 1 à 12.
